# EUROPÄISCHE PATENTSCHRIFT

(11) **EP 1 137 596 B1**
(45) Veröffentlichungstag und Bekanntmachung des Hinweises auf die Patenterteilung: **23.04.2003**
(21) Anmeldenummer: 99971784.6
(22) Anmeldetag: 10.11.1999
(51) Int. Cl.: C01G 31/00, B01J 23/68, C07C 51/265, C01G 1/00

(54) **SILBER- UND VANADIUMOXID ENTHALTENDES MULTIMETALLOXID UND DESSEN VERWENDUNG**
POLYMETAL OXIDE CONTAINING SILVER OXIDE AND VANADIUM OXIDE AND THE USE THEREOF
OXYDE POLYMETALLIQUE CONTENANT DE L'OXYDE D'ARGENT ET DE L'OXYDE DE VANADIUM, ET SON UTILISATION

(30) Priorität: 10.11.1998 DE 19851786
(43) Veröffentlichungstag der Anmeldung: 04.10.2001
(73) Patentinhaber: BASF AKTIENGESELLSCHAFT, 67056 Ludwigshafen (DE)
(72) Erfinder: HEIDEMANN, Thomas, D-69469 Weinheim (DE); HIBST, Hartmut, D-69198 Schriesheim (DE); BAUER, Stefan, D-67069 Ludwigshafen (DE); DIETRICH, Ulf, D-67269 Grünstadt (DE)
(86) Internationale Anmeldenummer: EP9908579
(87) Internationale Veröffentlichungsnummer: WO00027753

(56) Entgegenhaltungen:
- WO-A-98/37967
- DE-A- 19 705 326
- US-A- 5 695 892
- L. ZNAIDI, N. BAFFIER, M. HUBER: "Synthesis of Vanadium bronzes MxV2O5 through sol-gel processes 1. monoclinic bronzes (M = Na, Ag)" MATERIALS RESEARCH BULLETIN, Bd. 24, Nr. 12, 1989, Seiten 1501-1514, XP000115408
- ZHANG HUI-LIANG, ZHONG WEI, DUAN XIANG, FU XIAN-CAI: "A Study of catalytic activity, constituent and structure of V-Ag catalyst for selective Oxidation of toluene to benzaldehyde" JOURNAL OF CATALYSIS, Bd. 129, 1991, Seiten 426-437, XP002133159
- A. CASALOT, M. POUCHARD: "Sur quelques nouvelles phases non-stoechiometrique du systeme Ag2O-V2O5-VO2 : I. Etude chimique et cristallographique" BULL. SOC. CHIM. FR., Nr. 10, 1967, Seiten 3817-3820, XP002133176 in der Anmeldung erwähnt
- CHEMICAL ABSTRACTS, vol. 109, no. 18, 1988 Columbus, Ohio, US; abstract no. 157307, V. VOLKOV, B.G. GOLOVKIN: "Phase compositions of the silver vanadium oxide (Ag0.8V2O5)-vanadium pentoxide-copper vanadium oxide (CuV2O5) system" XP002133177 & ZH. NEORG. KHIM., Bd. 33, Nr. 7, 1988, Seiten 1833-1835,

## Beschreibung

Die Erfindung betrifft ein Multimetalloxid der allgemeinen Formel I

Ag_{a-b}M_{b}V₂Oₓ * c H₂O, I

in der M ein Metall ausgewählt aus der Gruppe Li, Na, K, Rb, Cs, Tl, Mg, Ca, Sr, Ba, Cu, Zn, Cd, Pb, Cr, Au, Al, Fe, Co, Ni und/oder Mo ist,
- a: einen Wert von 0,3 bis 1,9 und
- b: einen Wert von 0 bis 0,5 hat, mit der Maßgabe, daß die Differenz (a-b) ≥ 0,1 ist und
- c: einen Wert von 0 bis 20 hat und
- x: eine Zahl, die sich stöchiometrisch durch die Wertigkeit und Häufigkeit der von Sauerstoff verschiedenen Elemente in der Formel I bestimmt, bedeutet,
das in einer Kristallstruktur vorliegt, die ein Pulverröntgendiagramm ergibt, welches Beugungsreflexe bei den Netzebenenabständen d 15,23 ± 0,6, 12,16 ± 0,4, 10,68 ± 0,3, 3,41 ± 0,04, 3,09 ± 0,04, 3,02 ± 0,04, 2,36 ± 0,04 und 1,80 ± 0,04 Å hat.

Bekanntermaßen wird eine Vielzahl von Carbonsäuren und/oder Carbonsäureanhydriden technisch durch die katalytische Gasphasenoxidation von aromatischen Kohlenwasserstoffen, wie Benzol, o-, m- oder p-Xylol, Naphthalin, Toluol oder Durol (1,2,4,5-Tetramethylbenzol) in Festbettreaktoren, vorzugsweise Rohrbündelreaktoren, hergestellt. Dabei werden je nach Ausgangsmaterial beispielsweise Benzoesäure, Maleinsäureanhydrid, Phthalsäureanhydrid, Isophthalsäure, Terephthalsäure oder Pyromellithsäureanhydrid gewonnen. Dazu wird im allgemeinen ein Gemisch aus einem molekularen Sauerstoff enthaltenden Gas, beispielsweise Luft und das zu oxidierende Ausgangsmaterial durch eine Vielzahl in einem Reaktor angeordneter Rohre geleitet, in denen sich eine Schüttung mindestens eines Katalysators befindet. Zur Temperaturregelung sind die Rohre von einem Wärmeträgermedium, beispielsweise einer Salzschmelze, umgeben. Trotz dieser Thermostatisierung kann es in der Katalysatorschüttung zur Ausbildung sogenannter "Heißer Flecken" ("hot spots") kommen, in denen eine höhere Temperatur herrscht als im übrigen Teil der Katalysatorschüttung. Diese "hot spots" geben Anlaß zu Nebenreaktionen, wie der Totalverbrennung 5 des Ausgangsmaterials oder führen zur Bildung unerwünschter, vom Reaktionsprodukt nicht oder nur mit viel Aufwand abtrennbarer Nebenprodukte, beispielsweise zur Bildung von Phthalid oder Benzoesäure bei der Herstellung von Phthalsäureanhydrid (PSA) aus o-Xylol.

Zur Abschwächung dieses "hot spots" wurde in der Technik dazu übergegangen, unterschiedlich aktive Katalysatoren schichtweise in der Katalysatorschüttung anzuordnen, wobei in der Regel der weniger aktive Katalysator so im Festbett angeordnet ist, daß das Reaktionsgasgemisch mit ihm als erstes in Kontakt kommt, d.h. er liegt in der Schüttung zum Gaseintritt hin, wohingegen der aktivere Katalysator zum Gasaustritt aus der Katalysatorschüttung hin gelegen ist (DE-A 25 462 68, EP-A 28 64 48, DE-A 29 48 163, EP-A 16 32 31, US-A 46 65 200). Die unterschiedlich aktiven Katalysatoren in der Katalysatorschüttung können bei der gleichen Temperatur dem Reaktionsgas ausgesetzt werden, es können die beiden Schichten aus unterschiedlich aktiven Katalysatoren aber auch auf unterschiedliche Reaktionstemperaturen thermostatisiert mit dem Reaktionsgas in Kontakt gebracht werden (DE-A 28 30 765). Nach EP-A 16 32 31 können mehrere der genannten Maßnahmen zur Einstellung der beschriebenen Aktivitätsstrukturierung gleichzeitig angewendet werden. Die deutsche Patentanmeldung Aktenzeichen P 19 823 262 beschreibt eine Variante unter Einsatz von mehreren Katalysatoren, bei der die Aktivität der Katalysatoren von der Gaseintrittseite bis zur Gasaustrittseite quasi kontinuierlich zunimmt.

Um Verunreinigungen an störenden, farbgebenden Komponenten wie Phthalid bzw. Naphthochinon zu minimieren und so ein PSA guter Qualität zu erhalten und um Verunreinigungen des Abgases durch Rest-Xylol bzw. Rest-Naphthalin zu vermeiden, wird die Reaktion unter Vollumsatz (d.h. > 99,9 % Umsatz bezüglich des eingesetzten Eduktes) gefahren (K. Towae et al. in Ullmann's Encyclopedia of Industrial Chemistry, Vol. A20, 1992, 181). Eine umfassende Schilderung des Standes der Technik zur selektiven o-Xylol-Oxidation sowie bezüglich des Verfahrens und der Katalysatorherstellung wird in WO 98/37967 sowie von K. Towae et. al., siehe oben, gegeben.

In EP-A 256 352 wird eine besondere Verfahrensvariante zur Herstellung von PSA beschrieben, bei der zunächst o-Xylol in flüssiger Phase mit molekularem Sauerstoff an einem homogen gelösten Kobaltkatalysator zu Tolylsäure oxidiert wird und die entstandene Tolylsäure anschließend in der Gasphase an einem herkömmlichen Heterogenkatalysator zu PSA weiteroxidiert wird.

5 Als Katalysatoren haben sich für diese Oxidationsreaktionen sogenannte Schalenkatalysatoren bewährt, bei denen die katalytisch aktive Masse schalenförmig auf einem im allgemeinen unter den Reaktionsbedingungen inerten, nicht-porösen Trägermaterial, wie Quarz (SiO₂), Porzellan, Magnesiumoxid, Zinndioxid, Siliciumcarbid, Rutil, Tonerde (Al₂O₃), Aluminiumsilikat, Magnesiumsilikat (Steatit), Zirkoniumsilikat oder Cersilikat oder Mischungen dieser Trägermaterialien aufgebracht ist. Als katalytisch aktiver Bestandteil der katalytisch aktiven Masse dieser Schalenkatalysatoren dient im allgemeinen neben Titandioxid, in Form seiner Anatas-Modifikation, Vanadiumpentoxid. Des weiteren können in der katalytisch aktiven Masse in geringen Mengen eine Vielzahl anderer oxidischer Verbindungen enthalten sein, die als Promotoren die Aktivität und Selektivität des Katalysators beeinflussen, beispielsweise indem sie seine Aktivität absenken oder erhöhen. Als solche Promotoren seien beispielhaft die Alkalimetalloxide, insbesondere Lithium-, Kalium-, Rubidium- und Cäsiumoxid, Thallium(I)oxid, Aluminiumoxid, Zirkoniumoxid, Eisenoxid, Nickeloxid, Kobaltoxid, Manganoxid, Zinnoxid, Silberoxid, Kupferoxid, Chromoxid, Molybdänoxid, Wolframoxid, Iridiumoxid, Tantaloxid, Nioboxid, Arsenoxid, Antimonoxid, Ceroxid und Phosphorpentoxid genannt. Als die Aktivität vermindernder und die Selektivität erhöhender Promotor wirken z.B. die Alkalimetalloxide, wohingegen oxidische Phosphorverbindungen, insbesondere Phosphorpentoxid, die Aktivität des Katalysators erhöhen, aber dessen Selektivität vermindern.

EP-A 447 267 betrifft einen herkömmlichen V₂O₅-TiO₂(Anatas)-Katalysator zur Herstellung von Phthalsäureanhydrid, der neben anderen Dotierungskomponenten noch geringe Mengen an Silber enthalten kann.

Obwohl die Verfahren zur Oxidation von aromatischen Kohlenwasserstoffen zu Carbonsäuren und/oder Carbonsäureanhydriden, insbesondere die Oxidation von o-Xylol und/oder Naphthalin zu PSA, seit Jahrzehnten intensivst beforscht werden, besteht nach wie vor ein Bedarf an verbesserten Katalysatoren für diesen Zweck.

Silber-Vanadiumoxid-Verbindungen mit einem atomaren Ag/V-Verhältnis < 1 sind als Silber-Vanadiumoxid-Bronzen bekannt. Bei diesen handelt es sich um im allgemeinen halbleitende oder metallisch leitfähige, oxidische Festkörper, die bevorzugt Schicht- oder Tunnelstrukturen aufweisen, wobei das vanadium im [V₂O₅]_{∞}-Wirtsgitter teilweise reduziert zu V(IV) vorliegt. α-AgₓV₂O₅-Bronzen weisen eine orthorhombische Kristallstruktur auf. Sie enthalten anreduzierte [V₂O₅]_{∞}-Schichten parallel zur Netzebene (001), die aus kanten- und eckenverknüpften VO₅-Pyramiden bestehen. Die Ag-5 Kationen sind zwischen den anreduzierten [V₂O₅]_{∞}-Schichten eingelagert. Im Falle der β-AgₓV₂O₅-Bronzen mit x = 0,3-0,4 liegen Tunnelstrukturen vor. Das zugrunde liegende β-[V₂O₅]_{∞}-Wirtsgitter ist unter Ausbildung großer Kanäle aus hochverzerrten VO₆-Oktaedern und verzerrt trigonal-bipyramidalen VO₅-Einheiten aufgebaut. Die Ag-Kationen sind in den Kanälen des β-[V₂O₅]_{∞}-Wirtsgitters eingelagert. Dagegen enthält die idealisierte Struktur der Vanadiumbronze δ-AgₓV₂O₅ (x = 0,6-0,9) Schichten aus kantenverknüpften VO₆-Oktaedern, zwischen die die Ag-Kationen eingelagert sind.

Weitere Angaben zur Zusammensetzung und Kristallstruktur der oxidischen Bronzen sind beschrieben in A.F. Wells, Structural Inorganic Chemistry, Fifth Edition, Clarendon Press, Oxford, 1984, S. 621-625 und in C.N.R. Rao, B. Raveau, Transition Metal Oxides, VCH Publishers, Inc., New York, 1995, Seiten 176-179. Spezielle Angaben zur Darstellung und Struktur der AgₓV₂O₅-Bronzen finden sich in "Gmelin Handbuch der anorganischen Chemie", 8. Auflage, Silber, Teil B4, System-Nummer 61, Springer-Verlag, Berlin-Heidelberg-New-York, 1974, S. 274-277.

Aus EP-A 856490 ist ein spezielles Silber-Vanadium-Oxid und dessen Verwendung als Kathodenmaterial in elektrochemischen Zellen bekannt, das in einer Festkörperreaktion zwischen Silberoxid und einem Vanadiumoxid, wie V₂O₅ oder V₆O₁₃, bei Temperaturen von 500°C bis 520°C erzeugt wird.

Auch die Verwendung von Silber-Vanadiumoxid-Bronzen als Oxidationskatalysator ist bekannt. So beschreiben Y.I. Andreikov, A.A. Lyapkin und V.L. Volkov in Neftekhimiya 17, 559 (1977) die Verwendung von Ag-V₂O₅-Bronzen mit einem Molverhältnis Ag:V₂O₅ von 0,8:1 zur Oxidation von Toluol zu Benzaldehyd/Benzoesäure, wobei die Selektivität zu Wertprodukten mit zunehmendem Umsatz abnimmt. Diese Katalysatoren werden durch Zusammenschmelzen der Ausgangsmaterialien Silber oder Silbernitrat und V₂O₅ bei 750°C erhalten, wodurch ein 3-phasiges Gemisch entsteht, das aufgrund seiner Herstellungsweise eine geringe BET-Oberfläche hat. Zusätzlich können diese Katalysatoren Kupfer enthalten.In RU-PS 2088 567 werden von Y.I. Andreikov et. al. Ag-V₂O₅-Bronzen vorstehender Zusammensetzung auf verschiedenen Trägermaterialien zur Oxidation von Toluol zu Benzaldehyd und Benzoesäure eingesetzt. Nach den Angaben der Beispiele wird der höchste Umsatz bei Verwendung eines Katalysators erzielt, der die Ag-V₂O₅-Bronze schalenförmig auf einem Siliciumnitrid-Trägermaterial aufgebracht enthält. Dabei beträgt der Toluolumsatz zu Benzaldehyd und Benzoesäure bei 420°C insgesamt weniger 15 %. Diese Katalysatoren arbeiten folglich nicht wirtschaftlich.

Ferner beschreiben E.I. Andreikov und V. Volkov in Kinet. Katal. 22, 963 (1981) sowie 22, 1207 (1981) die selektive Oxidation von o-Xylol oder Naphthalin unter Verwendung von Ag-V₂O₅-Bronzen mit einem Molverhältnis Ag:V₂O₅ von 0 - 1:1, wobei ein Maximum bezüglich Aktivität/Selektivität im Bereich von 0,5 - 0,86:1 auftritt. Auch bei dieser Reaktion nimmt die Wertproduktselektivität mit zunehmendem Umsatz ab. Die in diesen Publikationen beschriebenen Katalysatoren werden ebenfalls durch Zusammenschmelzen der Ausgangsmaterialien erhalten.

Weiterhin ist aus JP-A 46-42883 (1971) die Oxidation von o-Xylol zu Phthalsäureanhydrid unter Verwendung Ag-V₂O₅-haltiger Katalysatoren mit einem Molverhältnis Ag:V₂O₅ von 0,01 - 1:1 unter Zusatz von Tl in einem Molverhältnis Tl:V₂O₅ von 0,01 - 1:1 bekannt. Mit diesem System werden zwar hohe Umsätze erreicht, die Wertproduktselektivität und Ausbeute sind aber unbefriedigend. Diese Katalysatoren werden durch Imprägnieren des Trägermaterials und anschließendes Trocknen und Calcinieren hergestellt.

JP-A 44-29045 (1969) beschreibt die Oxidation von Isobuten zu Methacrolein mittels Silbervanadat-Katalysatoren, wobei in diesen Katalysatoren das Molverhältnis Ag/V ≥ 1 ist.

Schließlich ist die partielle Gasphasenoxidation von Toluol mit Silber-Vanadiumoxid-Bronzen aus US-A 3 485 876, DE-A 12 94 951 und US-A 41 37 259 bekannt. Das Ag:V-Molverhältnis in diesen Katalysatoren liegt bei 1:1. Ebenso ist die partielle Gasphasenoxidation von Cyclopentadien an Ag-V₂O₅ (mit einem V:Ag-Molverhältnis = 1:0,003) bekannt (K.-W. Jun et al., Appl. Catal 63, 267-278 (1990)), wobei die Ag-V₂O₅-Katalysatoren nur V₂O₅ und keine anderen identifizierbaren Festkörperphasen aufweisen. Die selektive Oxidation von nicht-cyclischen, ungesättigten Kohlenwasserstoffen, insbesondere die Oxidation von 1,3-Butadien zu Furan, mit Hilfe von Silbervanadaten wird in DE-A 19705326 beschrieben.

In allen Fällen war die Selektivität und Ausbeute zur Herstellung der gewünschten Wertprodukte unbefriedigend, so daß eine technische Anwendung der Silber-Vanadiumoxid-Bronzen wirtschaftlich uninteressant war.

Es bestand daher die Aufgabe, neue Katalysatoren und Ausgangsverbindungen zu deren Herstellung für Verfahren zur Oxidation aromatischer Kohlenwasserstoffe sowie Verfahren zur Herstellung dieser Katalysatoren und Ausgangsverbindungen für diese Katalysatoren zur Verfügung zu stellen. Diese Katalysatoren sollten bezüglich Aktivität und Selektivität bei der Oxidation aromatischer Kohlenwasserstoffe zu Carbonsäuren oder Carbonsäureanhydriden, insbesondere bei der Oxidation von o-Xylol und/oder Naphthalin zu Phthalsäureanhydrid, verbesserte Eigenschaften verglichen mit bekannten Katalysatoren auf Ag-V₂O₅-Basis haben.

Dementsprechend wurden Multimetalloxide der allgemeinen Formel I

Ag_{a-b}M_{b}V₂Oₓ * c H₂O, I

in der M ein Metall ausgewählt aus der Gruppe Li, Na, K, Rb, Cs, Tl, Mg, Ca, Sr, Ba, Cu, Zn, Cd, Pb, Cr, Au, Al, Fe, Co, Ni und/oder Mo ist,
- a: einen Wert von 0,3 bis 1,9 und
- b: einen Wert von 0 bis 0,5 hat, mit der Maßgabe, daß die Differenz (a-b) ≥ 0,1 ist und
- c: einen Wert von 0 bis 20 hat und
- x: eine Zahl, die sich durch die Wertigkeit und Häufigkeit der von Sauerstoff verschiedenen Elemente in der Formel I bestimmt, bedeutet,
die in einer Kristallstruktur vorliegen, die ein Pulverröntgendiagramm ergibt, welches Beugungsreflexe bei den Netzebenenabständen d 15,23 ± 0,6, 12,16 ± 0,4, 10,68 ± 0,3, 3,41 ± 0,04, 3,09 ± 0,04, 3,02 ± 0,04, 2,36 ± 0,04 und 1,80 ± 0,04 Å hat, sowie ein Verfahren zu deren Herstellung gefunden.

Des weiteren wurden Präkatalysatoren für die Herstellung oder Erzeugung von Schalenkatalysatoren für die Gasphasenpartialoxidation von aromatischen Kohlenwasserstoffen mit einem molekularen Sauerstoff enthaltenden Gas gefunden, bestehend aus einem inerten, nicht-porösen Trägermaterial und einer oder mehreren darauf schalenförmig aufgebrachten Schichten, wobei diese schalenförmige Schicht oder Schichten 30 bis 100 Gew.-%, bezogen auf das Gesamtgewicht dieser Schicht oder Schichten, eines vorstehend genannten Multimetalloxids enthalten, sowie z.B. aus diesen Präkatalysatoren oder den erfindungsgemäßen Multimetalloxiden erhältliche Schalenkatalysatoren für die Gasphasenpartialoxidation von aromatischen Kohlenwasserstoffen, bestehend aus einem inerten, nicht-porösen Trägermaterial und einer oder mehreren darauf aufgebrachten, die katalytisch aktive Masse enthaltende Schicht oder Schichten, deren katalytisch aktive Masse, bezogen auf ihr Gesamtgewicht, 30 bis 100 Gew.-% einer oder mehrerer Silber-Vanadiumoxid-Bronzen mit einem Ag : V-Atomverhältnis von 0,15 bis 0,95 enthält und eine BET-Oberfläche von 2 bis 100 m²/g hat.

Außerdem wurde ein Verfahren zur Herstellung von Carbonsäuren und/oder Carbonsäureanhydriden durch die partielle Oxidation von aromatischen Kohlenwasserstoffen, insbesondere von o-Xylol oder Naphthalin oder Gemischen dieser Verbindungen oder von Toluol, in der Gasphase mit einem molekularen Sauerstoff enthaltenden Gas bei erhöhter Temperatur an einem Katalysator, dessen katalytisch aktive Masse auf einem inerten Trägermaterial schalenförmig aufgebracht ist, gefunden, das dadurch gekennzeichnet ist, daß man einen Schalenkatalysator, dessen katalytisch aktive Masse, bezogen auf ihr Gesamtgewicht, 30 bis 100 Gew.-% einer Silber-Vanadiumoxid-Bronze mit einem Ag:V-Atomverhältnis von 0,15 bis 0,95 enthält und eine BET-Oberfläche von 2 bis 100 m²/g hat, in Anoder Abwesenheit mindestens eines von diesem verschiedenen Schalenkatalysators zur Oxidation von aromatischen Kohlenwasserstoffen zu Carbonsäuren und/oder Carbonsäureanhydriden, der in seiner katalytisch aktiven Masse als wesentliche katalytisch aktive Bestandteile Vanadiumpentoxid und Anatas enthält, verwendet, und bei Anwesenheit eines solchen zweiten Schalenkatalysators, diesen in einer kombinierten Katalysatorschüttung mit dem Schalenkatalysator obenstehender Zusammensetzung im Oxidationsreaktor einsetzt.

Die Angabe der Röntgenbeugungsreflexe erfolgt in dieser Anmeldung in Form der von der Wellenlänge der verwendeten Röntgenstrahlung unabhängigen Netzebenenabstände d[Å], die sich aus dem gemessenen Beugungswinkel mittels der Bragg'schen Gleichung errechnen lassen.

In der Regel weist das vollständige Pulverröntgenbeugungsdiagramm des erfindungsgemäßen Multimetalloxids der Formel I unter anderem die in Tabelle 1 aufgelisteten 17 Beugungsreflexe auf. Weniger intensive Beugungsreflexe des Pulverröntgendiagramms der erfindungsgemäßen Multimetalloxide der Formel I wurden in Tabelle 1 nicht berücksichtigt.

**Tabelle 1:**

| Beugungsreflex | d [Å] |
|---|---|
| 1 | 15,23 ± 0,6 |
| 2 | 12,16 ± 0,4 |
| 3 | 10,68 ± 0,3 |
| 4 | 5,06 ± 0,06 |
| 5 | 4,37 ± 0,04 |
| 6 | 3,86 ± 0,04 |
| 7 | 3,41 ± 0,04 |
| 8 | 3,09 ± 0,04 |
| 9 | 3,02 ± 0,04 |
| 10 | 2,58 ± 0,04 |
| 11 | 2,48 ± 0,04 |
| 12 | 2,42 ± 0,04 |
| 13 | 2,36 ± 0,04 |
| 14 | 2,04 ± 0,04 |
| 15 | 1,93 ± 0,04 |
| 16 | 1,80 ± 0,04 |
| 17 | 1,55 ± 0,04 |

In Abhängigkeit vom Kristallinitätsgrad und der Texturierung der erhaltenen Kristalle des erfindungsgemäßen Multimetalloxids kann es allerdings zu einer Abschwächung der Intensität der Beugungsreflexe im Pulverröntgendiagramm kommen, die soweit gehen kann, daß einzelne intensitätschwächere Beugungsreflexe im Pulverröntgendiagramm nicht mehr detektierbar sind, ohne daß sich dies auf die Eigenschaften der aus dem erfindungsgemäßen Multimetalloxid hergestellten Präkatalysatoren und Katalysatoren nachteilig auswirkt. Das Fehlen einzelner intensitätsschwächerer Beugungsreflexe im Pulverröntgendiagramm bei einem Multimetalloxid der chemischen Zusammensetzung gemäß Formel I bedeutet somit nicht, daß ein nicht-erfindungsgemäßes Multimetalloxid vorliegt, hingegen ist das Vorliegen sämtlicher 17 Beugungsreflexe im Pulverröntgendiagramm ein Indiz dafür, daß es sich dabei um ein erfindungsgemäßes Multimetalloxid besonders hoher Kristallinität handelt. Ein hoher Kristallinitätsgrad der erfindungsgemäßen Multimetalloxide kann sich vorteilhaft auf deren Verarbeitungseigenschaften bei der Herstellung der erfindungsgemäßen Präkatalysatoren und Katalysatoren auswirken. Es versteht sich für den Fachmann von selbst, daß Mischungen der erfindungsgemäßen Multimetalloxide mit anderen kristallinen Verbindungen zusätzliche Beugungsreflexe aufweisen. Solche Mischungen des Multimetalloxids mit anderen kristallinen Verbindungen können gezielt durch Vermischen des erfindungsgemäßen Multimetalloxids mit solchen Verbindungen hergestellt werden oder können bei der Präparation der erfindungsgemäßen Multimetalloxide durch nicht vollständige Umsetzung der Ausgangsmaterialien entstehen.

Die Beugungsreflexe 1 bis 17 gemäß Tabelle 1 haben im allgemeinen die in Tabelle 2 angegebenen ungefähren relativen Intensitäten (Iᵣₑₗ [%]):

**Tabelle 2**

| Beugungsreflex | Iᵣₑₗ [%] |
|---|---|
| 1 | 16 |
| 2 | 11 |
| 3 | 18 |
| 4 | 11 |
| 5 | 23 |
| 6 | 16 |
| 7 | 80 |
| 8 | 61 |
| 9 | 100 |
| 10 | 23 |
| 11 | 24 |
| 12 | 23 |
| 13 | 38 |
| 14 | 26 |
| 15 | 31 |
| 16 | 43 |
| 17 | 36 |

Entsprechend den vorstehenden Ausführungen zur Intensität der Beugungsreflexe können die in Tabelle 2 angegebenen 17 Intensitätswerte in ihrer Relation zueinander schwanken.

Im Multimetalloxid der Formel I kann der Wert der Variablen a 0,3 bis 1,9, vorzugsweise 0,5 bis 1,0 und besonders bevorzugt 0,6 bis 0,9 betragen und der Wert der Variablen b bei 0 bis 0,5, vorzugsweise bei 0 bis 0,3, insbesondere bei 0 bis 0,1 liegen, wobei die Maßgabe gilt, daß die Differenz (a-b) größer oder gleich 0,1 ist. Die Zahl x bestimmt sich aus der Wertigkeit und Häufigkeit der von Sauerstoff verschiedenen Elemente im Multimetalloxid der Formel I. Die Zahl c, die ein Maß für den Wassergehalt ist, kann 0 bis 20, vorzugsweise 0 bis 5, insbesondere 0 bis 1 betragen.

Besonders bevorzugte Multimetalloxide der Formel I haben eine Zusammensetzung der Formel

AgₐV₂Oₓ* c H₂O,

worin a einen Wert von 0,6 bis 0,9 hat, der Wert der Variablen x sich aus der Häufigkeit und Wertigkeit der Silber- und Vanadiumkomponente bestimmt und c einen Wert von 0 bis 5 hat.

Bei den erfindungsgemäßen Multimetalloxiden handelt es sich um neue chemische Verbindungen.

Die neuen Multimetalloxide haben im allgemeinen eine faserförmige Kristallmorphologie, wobei das mittlere Verhältnis aus Faserdurchmesser zu Faserlänge < 0,6, bevorzugt < 0,3 und besonders bevorzugt < 0,1 beträgt, wobei dieses Verhältnis selbstverständlich immer > 0 ist. Die spezifische Oberfläche nach BET, gemessen gemäß DIN 66 131, die auf den "Recommendations 1984" der IUPAC International Union of Pure and Applied Chemistry (s. Pure & Appl. Chem. 57, 603 (1985)) basiert, beträgt in der Regel mehr als 1 m²/g, bevorzugt 3 bis 250 m²/g, insbesondere 10 bis 250 m²/g und besonders bevorzugt 20 bis 80 m²/g.

Als Metalle M können die Metalle Li, Na, K, Rb, Cs, Tl, Mg, Ca, Sr, Ba, Cu, Zn, Cd, Pb, Cr, Au, Al, Fe, Co, Ni und/oder Mo Bestandteile der erfindungsgemäßen Multimetalloxide sein, bevorzugt sind Na, K, Rb, Tl, Au und Cu.

Zur Herstellung der erfindungsgemäßen Multimetalloxide wird im allgemeinen eine Suspension von Vanadiumpentoxid (V₂O₅) mit der Lösung eines Silbersalzes in einem Lösungsmittel und gegebenenfalls einer Lösung einer Verbindung der Metallkomponente M erhitzt. Als Lösungsmittel für diese Umsetzung können polare organische Lösungsmittel, wie Polyole, Polyether oder Amine, z.B. Pyridin, dienen, bevorzugt wird als Lösungsmittel Wasser verwendet. Als Silbersalz wird bevorzugt Silbernitrat verwendet, die Verwendung anderer löslicher Silbersalze, z.B. Silberacetat, Silberperchlorat oder Silberfluorid ist ebenfalls möglich. Als Salze der Metallkomponente M werden in der Regel solche gewählt, die im verwendeten Lösungsmittel löslich sind. Wird Wasser als Lösungsmittel bei der Herstellung der erfindungsgemäßen Multimetalloxide verwendet, können beispielsweise die Perchlorate oder Carboxylate, insbesondere die Acetate, der Metallkomponente M eingesetzt werden, bevorzugt werden die Nitrate der betreffenden Metallkomponente M verwendet.

Die Umsetzung des V₂O₅ mit dem Silbersalz und gegebenenfalls dem Salz der Metallkomponente M kann im allgemeinen bei Raumtemperatur oder bei erhöhter Temperatur durchgeführt werden. In der Regel wird die Umsetzung bei Temperaturen von 20 bis 375°C, vorzugsweise bei 20 bis 100°C und besonders bevorzugt bei 60 bis 100°C vorgenommen. Liegt die Temperatur der Umsetzung oberhalb der Temperatur des Siedepunktes des verwendeten Lösungsmittels, wird die Umsetzung zweckmäßigerweise unter dem Eigendruck des Reaktionssystems in einem Druckgefäß ausgeführt, vorzugsweise werden die Reaktionsbedingungen so gewählt, daß die Umsetzung bei Atmosphärendruck durchgeführt werden kann. Die Dauer dieser Umsetzung kann in Abhängigkeit von der Art der umgesetzten Ausgangsmaterialien und den angewandten Temperaturbedingungen 10 Minuten bis 3 Tage betragen. Eine Verlängerung der Reaktionszeit der Umsetzung, beispielsweise auf 5 Tage und mehr, ist möglich. In der Regel wird die Umsetzung des V₂O₅ mit dem Silbersalz und gegebenenfalls einem oder mehreren Salzen der Metallkomponente M zum erfindungsgemäßen Multimetalloxid während eines Zeitraums von 6 bis 24 Stunden durchgeführt.

Bei der Umsetzung verändert sich die orangerote Farbe der V₂O₅-Suspension und es bildet sich die neue Verbindung in Form einer dunkelbraunen Suspension.

Je nach der gewünschten chemischen Zusammensetzung des Multimetalloxids der Formel I werden zu dessen Herstellung die sich aus a und b von Formel I ergebenden Mengen von V₂O₅, Silbersalz und gegebenenfalls dem Salz der Metallkomponente M miteinander umgesetzt. So wird im allgemeinen das Silbersalz mit dem Vanadiumpentoxid in einem Mengenverhältnis umgesetzt, das einem Atomverhältnis Ag : V von 0,15 bis 0,95, vorzugsweise von 0,25 bis 0,5 entspricht, entsprechend einem Wert für a in Formel I von 0,3 bis 1,9 bzw. 0,5 bis 1,0. Besonders bevorzugt wird das Silbersalz bezüglich des Vanadiumpentoxids in einer Menge zugesetzt, die einem Atomverhältnis Ag : V von 0,3 bis 0,45 entspricht, entsprechend einem Wert für a in Formel I von 0,6 bis 0,9. Nach beendeter Umsetzung wird dabei das erfindungsgemäße Multimetalloxid mit faserförmiger Kristallmorphologie erhalten.

Das so gebildete erfindungsgemäße Multimetalloxid kann aus der Reaktionsmischung isoliert und bis zur weiteren Verwendung gelagert werden. Die Isolierung des Multimetalloxids kann z.B. durch Abfiltrieren der Suspension und Trocknen des erhaltenen Feststoffs erfolgen, wobei die Trocknung sowohl in herkömmlichen Trocknern, aber auch z.B. in Gefriertrocknern durchgeführt werden kann. Besonders vorteilhaft wird die Trocknung der erhaltenen Multimetalloxid-Suspension mittels Sprühtrocknung durchgeführt.

Es kann vorteilhaft sein, das bei der Umsetzung erhaltene Multimetalloxid vor dessen Trocknung salzfrei zu waschen. Die Sprühtrocknung wird im allgemeinen unter Atmosphärendruck oder vermindertem Druck vorgenommen. Je nach angewandtem Druck und verwendetem Lösungsmittel bestimmt sich die Eingangstemperatur des Trocknungsgases - im allgemeinen wird als solches Luft verwendet, es können aber selbstverständlich auch andere Trocknungsgase wie Stickstoff oder Argon, benutzt werden. Die Eingangstemperatur des Trocknungsgases in den Sprühtrockner wird vorteilhaft so gewählt, daß die Ausgangstemperatur des durch Verdampfung des Lösungsmittels abgekühlten Trocknungsgases 200°C für einen längeren Zeitraum nicht übersteigt. In der Regel wird die Ausgangstemperatur des Trocknungsgases auf 50 bis 150°C, vorzugsweise 100 bis 140°C eingestellt. Falls eine Lagerung des Multimetalloxids nicht beabsichtigt ist, kann die erhaltene Multimetalloxid-Suspension auch ohne vorherige Isolierung und Trocknung des Multimetalloxids der weiteren Verwendung zugeführt werden, beispielsweise zur Beschichtung der erfindungsgemäßen Präkatalysatoren.

Die erfindungsgemäßen Multimetalloxide werden als Vorläuferverbindung zur Herstellung der katalytisch aktiven Masse von Schalenkatalysatoren, wie sie zur Gasphasenoxidation von aromatischen Kohlenwasserstoffen zu Carbonsäuren und/oder Carbonsäureanhydriden mit einem molekularen Sauerstoff enthaltenden Gas eingesetzt werden, verwendet. Besonders vorteilhaft erweisen sich zu diesem Zweck Multimetalloxide gemäß Formel I, die eine faserförmige Kristallmorphologie mit einem mittleren Verhältnis aus Faserdurchmesser zu Faserlänge von kleiner 0,6, vorzugsweise von kleiner 0,3 und besonders bevorzugt von kleiner 0,1 haben, wobei dieses Verhältnis immer größer als 0 ist. Von diesen Multimetalloxiden faserförmiger Kristallmorphologie sind zu diesem Zweck wiederum solche bevorzugt, deren BET-Oberfläche 3 bis 250 m²/g, insbesondere 10 bis 250 m²/g und besonders bevorzugt 20 bis 80 m²/g beträgt. Bezüglich ihrer chemischen Zusammensetzung werden zur Herstellung der genannten Schalenkatalysatoren besonders bevorzugt Multimetalloxide gemäß Formel I verwendet, in denen a einen Wert von 0,6 bis 0,9 hat und b, von technisch praktisch unvermeidbaren und technisch unwirksamen, durch die verwendeten Ausgangsmaterialien eingeschleppten Verunreinigungen abgesehen, gleich 0 ist, insbesondere solche der Formel

AgₐV₂Oₓ* c H₂O,

in der a einen Wert von 0,6 bis 0,9 hat, c einen Wert von 0 bis 5 hat und x eine Zahl ist, die sich durch die Wertigkeit und Häufigkeit des Silbers und des Vanadiums in diesem Multimetalloxid bestimmt.

Auch wenn die erfindungsgemäßen Multimetalloxide vorzugsweise für die Herstellung der erfindungsgemäßen Schalenkatalysatoren zur Oxidation aromatischer Kohlenwasserstoffe eingesetzt werden, können sie auch als Vorläuferverbindung zur Herstellung herkömmlicher Trägerkatalysatoren oder von Vollkatalysatoren, also Katalysatoren die kein Trägermaterial enthalten, verwendet werden. Eine weitere Einsatzmöglichkeit für die erfindungsgemäßen Multimetalloxide besteht in deren Verwendung als Kathodenmaterial oder zur Herstellung von Kathodenmaterial für elektrochemische Zellen, beispielsweise Batterien.

Die Herstellung der erfindungsgemäßen Schalenkatalysatoren zur partiellen Oxidation aromatischer Kohlenwasserstoffe zu Carbonsäuren und/oder Carbonsäureanhydriden aus den erfindungsgemäßen Multimetalloxiden erfolgt zweckmäßigerweise über die Stufe eines sogenannten "Präkatalysators", der als solcher gelagert und gehandelt werden kann und aus dem der erfindungsgemäße Schalenkatalysator entweder durch thermische Behandlung hergestellt oder in situ im Oxidationsreaktor unter den Bedingungen der Oxidationsreaktion erzeugt werden kann. Bei dem Präkatalysator handelt es sich somit um eine Vorstufe des fertigen Schalenkatalysators, bestehend aus einem unter den Bedingungen der Präkatalysator- und Schalenkatalysator-Herstellung als auch unter den Bedingungen der partiellen Oxidation von aromatischen Kohlenwasserstoffen zu Carbonsäuren und/oder Carbonsäureanhydriden inerten, nicht-porösen Trägermaterial und einer oder mehreren darauf schalenförmig aufgebrachten Schichten, wobei diese schalenförmige Schicht oder Schichten des Präkatalysators 30 bis 100 Gew.-%, vorzugsweise 50 bis 100 Gew.-%, bezogen auf das Gesamtgewicht dieser Schicht oder Schichten, eines Multimetalloxids gemäß Formel I enthalten. Besonders bevorzugt besteht die schalenförmige Schicht oder Schichten vollständig aus einem Multimetalloxid gemäß Formel I. Enthält die katalytisch aktive Schicht oder Schichten außer dem Multimetalloxid gemäß Formel I noch weitere Komponenten, können dies z.B. Inertmaterialien des Standes der Technik, wie Siliciumcarbid oder Steatit, oder aber auch nicht-erfindungsgemäße Katalysatoren zur Oxidation von aromatischen Kohlenwasserstoffen zu Carbonsäuren und/oder Carbonsäureanhydriden auf Vanadiumpentoxid/Anatas-Basis sein, wie sie z. B. eingangs bei der Schilderung des Standes der Technik erwähnt wurden.

Als inertes, nicht-poröses Trägermaterial für die erfindungsgemäßen Präkatalysatoren und Schalenkatalysatoren können praktisch alle Trägermaterialien des Standes der Technik, wie sie vorteilhaft bei der Herstellung von Schalenkatalysatoren für die Oxidation aromatischer Kohlenwasserstoffe zu Carbonsäuren und/oder Carbonsäureanhydriden eingesetzt werden, Verwendung finden, beispielsweise Quarz (SiO₂), Porzellan, Magnesiumoxid, Zinndioxid, Siliciumcarbid, Rutil, Tonerde (Al₂O₃), Aluminiumsilikat, Steatit (Magnesiumsilikat), Zirkoniumsilikat, Cersilikat oder Mischungen dieser Trägermaterialien. Der Ausdruck "nicht-porös" ist dabei im Sinne von "bis auf technisch unwirksame Mengen an Poren nicht-porös" zu verstehen, da technisch unvermeidlich eine geringe Anzahl Poren im Trägermaterial, das idealerweise keine Poren enthalten sollte, vorhanden sein können. Als vorteilhafte Trägermaterialien sind insbesondere Steatit und Siliciumcarbid hervorzuheben. Die Form des Trägermaterials ist für die erfindungsgemäßen Präkatalysatoren und Schalenkatalysatoren im allgemeinen nicht kritisch. Beispielsweise können Katalysatorträger in Form von Kugeln, Ringen, Tabletten, Spiralen, Röhren, Extrudaten oder Splitt verwendet werden. Die Dimensionen dieser Katalysatorträger entsprechen denen üblicherweise zur Herstellung von Schalenkatalysatoren für die Gasphasenpartialoxidation von aromatischen Kohlenwasserstoffen verwendeten Katalysatorträgern. Wie erwähnt, können die vorstehend genannten Trägermaterialien in Pulverform auch der katalytisch aktiven Masse der erfindungsgemäßen Schalenkatalysatoren zugemischt werden.

Zur schalenförmigen Beschichtung des inerten Trägermaterials mit dem erfindungsgemäßen Multimetalloxid können im Prinzip bekannte Methoden des Standes der Technik angewandt werden. Beispielsweise kann die bei der Umsetzung des Vanadiumpentoxids mit einem Silbersalz und gegebenenfalls einem oder mehreren Salzen der Metallkomponente M erhaltene Suspension gemäß den Verfahren von DE-A 1692938 und DE-A 1769998 in einer beheizten Dragiertrommel bei erhöhter Temperatur auf den aus inertem Trägermaterial bestehenden Katalysatorträger aufgesprüht werden, bis die gewünschte Menge an Multimetalloxid, bezogen auf das Gesamtgewicht des Präkatalysators erreicht ist. Anstelle von Dragiertrommeln können analog zu DE-A 2106796 auch Wirbelbettbeschichter, wie sie in DE-A 1280756 beschrieben sind, zur schalenförmigen Aufbringung des erfindungsgemäßen Multimetalloxids auf den Katalysatorträger eingesetzt werden. Anstelle der bei der Umsetzung des Vanadiumpentoxids mit einem Silbersalz und gegebenenfalls einem oder mehreren Salzen der Metallkomponente M erhaltenen Suspension des erfindungsgemäßen Multimetalloxids, kann, besonders bevorzugt, eine Aufschlämmung des nach Isolierung und Trocknung erhaltenen Pulvers des erfindungsgemäßen Multimetalloxids bei diesen Beschichtungsverfahren verwendet werden. Analog EP-A 744214 können der Suspension des erfindungsgemäßen Multimetalloxids, wie sie bei dessen Herstellung entsteht, oder einer Aufschlämmung eines Pulvers des erfindungsgemäßen, getrockneten Multimetalloxids in Wasser, einem organischen Lösungsmittel, wie höheren Alkoholen, mehrwertigen Alkoholen, z.B. Ethylenglykol, 1,4-Butandiol oder Glycerin, Dimethylformamid, Dimethylacetamid, Dimethylsulfoxid, N-Methylpyrrolidon oder cyclischen Harnstoffen, wie N,N'-Dimethylethylenharnstoff oder N,N'-Dimethylpropylenharnstoff, oder in Mischungen dieser organischen Lösungsmittel mit Wasser, organische Bindemittel, bevorzugt Copolymere, gelöst oder vorteilhaft in Form einer wässrigen Dispersion zugesetzt werden, wobei im allgemeinen Bindemittelgehalte von 10 bis 20 Gew.-%, bezogen auf den Feststoffgehalt der Suspension oder Aufschlämmung des erfindungsgemäßen Multimetalloxids angewandt werden. Geeignete Bindemittel sind z.B. Vinylacetat/Vinyllaurat-, Vinylacetat/Acrylat-, Styrol/Acrylat-, Vinylacetat/Maleat- oder Vinylacetat/Ethylen-Copolymere. Werden als Bindemittel organische Copolymer-Polyester, z.B. auf Basis von Acrylat/Dicarbonsäureanhydrid/Alkanolamin, in einer Lösung in einem organischen Lösungsmittel der Aufschlämmung des erfindungsgemäßen Multimetalloxids zugesetzt, kann analog zur Lehre der deutschen Patentanmeldung Aktenzeichen P 19823262.4 der Gehalt an Bindemittel auf 1 bis 10 Gew.-%, bezogen auf den Feststoffgehalt der Suspension oder Aufschlämmung, verringert werden.

Bei der Beschichtung des Katalysatorträgers mit den erfindungsgemäßen Multimetalloxiden werden im allgemeinen Beschichtungstemperaturen von 20 bis 500°C angewandt, wobei die Beschichtung in der Beschichtungsapparatur unter Atmosphärendruck oder unter reduziertem Druck erfolgen kann. Zur Herstellung der erfindungsgemäßen Präkatalysatoren wird die Beschichtung im allgemeinen bei 0°C bis 200°C, vorzugsweise bei 20 bis 150°C, insbesondere bei Raumtemperatur bis 100°C durchgeführt. Bei der Beschichtung des Katalysatorträgers mit einer feuchten Suspension der erfindungsgemäßen Multimetalloxide kann es zweckmäßig sein, höhere Beschichtungstemperaturen, z. B. Temperaturen von 200 bis 500°C, anzuwenden. Bei den vorstehend genannten tieferen Temperaturen kann bei Verwendung eines polymeren Bindemittels bei der Beschichtung ein Teil des Bindemittels in der auf dem Katalysatorträger aufgetragenen Schicht verbleiben.

Bei einer späteren Umwandlung des Präkatalysators in einen erfindungsgemäßen Schalenkatalysator durch thermische Behandlung bei Temperaturen über 200°C bis 500°C entweicht das Bindemittel durch thermische Zersetzung und/oder Verbrennung aus der aufgetragenen Schicht. Die Umwandlung des Präkatalysators in einen erfindungsgemäßen Schalenkatalysator kann auch durch thermische Behandlung bei Temperaturen über 500°C erfolgen, beispielsweise bei Temperaturen bis 650°C, vorzugsweise wird die thermische Behandlung bei Temperaturen von über 200°C bis 500°C, insbesondere bei 300 bis 450°C durchgeführt.

Wie im Folgenden noch näher ausgeführt werden wird, können sich die erfindungsgemäßen Multimetalloxide oberhalb 200°C, insbesondere bei Temperaturen von mehr als 300°C unter Ausbildung von Silber-Vanadiumoxid-Bronzen zersetzen, die Bestandteile der katalytisch aktiven Masse der erfindungsgemäßen Schalenkatalysatoren sind. Dementsprechend kann bei Beschichtungstemperaturen oberhalb von 200°C je nach den angewandten Bedingungen bereits ein Teil der auf den Katalysatorträger aufgetragenen erfindungsgemäßen Multimetalloxide zu katalytisch aktiven Silber-Vanadiumoxid-Bronzen und/oder bezüglich ihrer Struktur kristallographisch nicht aufgeklärten Silber-Vanadiumoxid-Verbindungen, die in die genannten Silber-Vanadiumoxid-Bronzen umgewandelt werden können, zersetzt werden. Diese Zersetzung geht in diesem Temperaturbereich allerdings sehr langsam vonstatten, so daß bei den im Bereich von oberhalb 200 bis 300°C beschichteten Katalysatorträgern die aufgebrachte Schicht im wesentlichen aus dem erfindungsgemäßen Multimetalloxid besteht,wie durch eine röntgenstrukturanalytische Untersuchung einer Abriebprobe der aufgebrachten Schicht festgestellt werden kann. Bei Beschichtungstemperaturen von 300 bis 500°C läuft diese Zersetzung praktisch vollständig ab, so daß bei einer Beschichtung bei 300 bis 500°C der erfindungsgemäße Schalenkatalysator ohne Durchlaufen der Vorstufe des Präkatalysators erhalten werden kann. Wird die Beschichtung des Katalysatorträgers mit den erfindungsgemäßen Multimetalloxiden im Temperaturbereich von oberhalb 200°C bis 300°C vorgenommen, enthält die aufgetragene Schicht in der Regel und abhängig von der Art des verwendeten Multimetalloxids und der zur Durchführung der Beschichtung benötigten Zeit unterschiedliche Mengen sowohl des erfindungsgemäßen Multimetalloxids als auch der durch dessen teilweise Zersetzung entstandenen Silber-Vanadiumoxid-Bronzen und/oder bezüglich ihrer Struktur kristallographisch nicht aufgeklärten Silber-Vanadiumoxid-Verbindungen.

Prinzipiell kann jede der vorstehend geschilderten Beschichtungsmethoden zur Herstellung der erfindungsgemäßen Präkatalysatoren bzw. der erfindungsgemäßen Schalenkatalysatoren angewandt werden. Besonders vorteilhafte Präkatalysatoren und Schalenkatalysatoren werden jedoch erhalten, wenn die erfindungsgemäßen Präkatalysatoren unter Berücksichtigung der vorstehenden Erläuterungen in Analogie zu den Katalysatorherstellverfahren von EP-A 714700 und WO 98/37967 durch Beschichten des inerten Katalysatorträgers mit einem, vorzugsweise sprühgetrockneten, Pulver des erfindungsgemäßen Multimetalloxids, besonders bevorzugt mit einem Multimetalloxid mit den vorstehend genannten vorteilhaften Eigenschaften hinsichtlich seiner BET-Oberfläche, Kristallmorphologie und chemischen Zusammensetzung, bei 0°C bis 200°C, vorzugsweise 20 bis 150°C, insbesondere bei Raumtemperatur bis 100°C, gegebenenfalls unter Zusatz eines der genannten Bindemittel, hergestellt werden.

Abriebproben dieser erfindungsgemäß aus dem Multimetalloxid gemäß Formel I hergestellten Präkatalysatoren weisen in ihrem Röntgenbeugungsdiagramm Beugungsreflexe u.a. bei den Netzebenenabständen d auf, wie sie vorstehend für die erfindungsgemäßen Multimetalloxide in Tabelle 1 angegeben worden sind.

Die erfindungsgemäßen Schalenkatalysatoren werden bevorzugt aus den erfindungsgemäßen Präkatalysatoren hergestellt oder aus diesen Präkatalysatoren im Reaktor für die Oxidation der aromatischen Kohlenwasserstoffe in situ erzeugt.

Bei der thermischen Behandlung der erfindungsgemäßen Präkatalysatoren bei Temperaturen von über 200 bis 650°C, vorzugsweise bei über 250°C bis 500°C, insbesondere bei 300 bis 450°C, die einer Temperung entspricht, werden diese in die erfindungsgemäßen Schalenkatalysatoren für die Gasphasenoxidation von aromatischen Kohlenwasserstoffen zu Carbonsäuren und/oder Carbonsäureanhydriden umgewandelt. Dabei zersetzen sich das oder gegebenenfalls die im Präkatalysator enthaltenen erfindungsgemäßen Multimetalloxide am Ende zu bereits bekannten und charakterisierten Silber-Vanadiumoxid-Bronzen (s. Bull. Soc. Chim. France 3817, 1967). Dies kann durch Röntgenbeugungsdiagramme von Abriebproben der katalytisch aktiven Schicht der durch die genannte thermische Behandlung des Präkatalysators erhaltenen, erfindungsgemäßen Schalenkatalysatoren festgestellt werden. Diese Umwandlung der im Präkatalysator enthaltenen erfindungsgemäßen Multimetalloxide zu bekannten Silber-Vanadiumoxid-Bronzen findet insbesondere auch in situ im Reaktor zur Gasphasenpartialoxidation von aromatischen Kohlenwasserstoffen zu Carbonsäuren und/oder Carbonsäureanhydriden, beispielsweise im Reaktor zur Herstellung von Phthalsäureanhydrid aus o-Xylol und/oder Naphthalin, bei den dabei im allgemeinen angewandten Temperaturen von 300 bis 450°C statt, wenn man anstelle des erfindungsgemäßen Schalenkatalysators einen erfindungsgemäßen Präkatalysator bei dieser Umsetzung einsetzt. Bis zum Ende der Umwandlung des erfindungsgemäßen Multimetalloxids zu den bekannten Silber-Vanadium-Bronzen ist dabei in der Regel ein steter Anstieg der Selektivität des Schalenkatalysators zu beobachten. Die dabei entstehenden Silber-Vanadiumoxid-Bronzen sind somit ein katalytisch aktiver Bestandteil der katalytisch aktiven Schicht des erfindungsgemäßen Schalenkatalysators.

Die thermische Umwandlung der erfindungsgemäßen Multimetalloxide zu Silber-Vanadiumoxid-Bronzen verläuft über eine Reihe von Reduktions- und Oxidationsreaktionen, die im einzelnen noch nicht verstanden sind. Es wurde festgestellt, daß abhängig von der Zusammensetzung der Atmosphäre, in der diese Umwandlung vorgenommen wird, an Sauerstoff, Inertgasen oder reduzierenden Gasen, und von der angewandten Temperatur, sowie weiterhin abhängig davon, ob der Präkatalysator ein organisches Bindemittel enthält oder nicht, als auch von der Art und Menge dieses Bindemittels, bei der thermischen Behandlung des Präkatalysators oder bei der Beschichtung des Trägermaterials bei erhöhter Temperatur aus den erfindungsgemäßen Multimetalloxiden zunächst andere Silber-Vanadiumoxid-Verbindungen als die vorgenannten Silber-Vanadiumoxid-Bronzen entstehen können, deren kristallographische Struktur nicht aufgeklärt ist, die sich aber unter den Bedingungen des Verfahrens zur Oxidation aromatischer Kohlenwasserstoffe zu Carbonsäureanhydriden und/oder Carbonsäuren im Reaktor in die genannten Silber-Vanadiumoxid-Bronzen mit den genannten Eigenschaften umwandeln, wie aus den Röntgenbeugungsdiagrammen von Abriebproben ausgebauter Katalysatoren festgestellt werden kann. Nach den vorliegenden Erkenntnissen ist dieser Vorgang reversibel, d.h. die in der katalytisch aktiven Masse des erfindungsgemäßen Schalenkatalysators enthaltene Silber-Vanadiumoxid-Bronze, kann z. B. nach dessen Ausbau aus dem Reaktor unter oxidierenden Bedingungen in eine andere Silber-Vanadiumoxid-Verbindung überführt werden, die nach erneutem Einbau des Katalysators in den Reaktor wieder zur betreffenden Silber-Vanadiumoxid-Bronze reduziert wird.

Für die Herstellung des erfindungsgemäßen Schalenkatalysators durch die Beschichtung eines inerten, nicht-porösen Katalysatorträgers mit dem erfindungsgemäßen Multimetalloxid bei Temperaturen von über 200 bis 500°C oder durch die thermische Behandlung des erfindungsgemäßen Präkatalysatoren bei oberhalb 200 bis 650°C bedeutet dies, daß die Erzeugung der die Silber-Vanadiumoxid-Bronze enthaltenden katalytisch-aktiven Masse in einer oder mehreren Stufen erfolgen kann. Die einstufige Erzeugung der Silber-Vanadiumoxid-Bronze in der katalytisch aktiven Schicht des erfindungsgemäßen Schalenkatalysators wird vorzugsweise durch die Behandlung des erfindungsgemäßen Präkatalysators unter den Bedingungen der Oxidation von aromatischen Kohlenwasserstoffen zu Carbonsäureanhydriden und/oder Carbonsäuren in situ im Oxidationsreaktor durchgeführt. Die einstufige Erzeugung der Silber-Vanadiumoxid-Bronze in der katalytisch aktiven Schicht der erfindungsgemäßen Schalenkatalysatoren kann aber auch außerhalb des Oxidationsreaktors erfolgen, z. B. bei der Beschichtung des Trägermaterials mit dem erfindungsgemäßen Multimetalloxid bei Temperaturen von oberhalb 200°C bis 500°C oder einer separaten thermischen Behandlung bei oberhalb 200 bis 650°C des bei Temperaturen von 0°C bis 200°C mit dem Multimetalloxid beschichteten Präkatalysators, wobei die obengenannten Einflußgrößen, wie die Zusammensetzung der Gasatmosphäre, An- oder Abwesenheit eines Bindemittels sowie Art und Menge des Bindemittels, zu berücksichtigen sind. Bei einer solchen Vorgehensweise werden zweckmäßigerweise im Einzelfall die optimalen Bedingungen für die Erzeugung der Silber-Vanadiumoxid-Bronze in der katalytisch aktiven Schicht des erfindungsgemäßen Schalenkatalysators in einem Vorversuch ermittelt.

Für die mehrstufige Erzeugung der Silber-Vanadiumoxid-Bronze in der katalytisch aktiven Schicht der erfindungsgemäßen Schalenkatalysatoren stehen eine Reihe von Vorgehensweisen zur Verfügung. Es kann z. B. ein bei Temperaturen von 0 bis 200°C mit dem erfindungsgemäßen Multimetalloxid beschichteter Präkatalysator unter für die Erzeugung der Silber-Vanadiumoxid-Bronze nicht optimierten Bedingungen einer thermischen Behandlung bei oberhalb 200 bis 650°C unterworfen werden, so daß sich aus dem Multimetalloxid die vorgenannten, bezüglich ihrer kristallographischen Struktur nicht aufgeklärten Silber-Vanadiumoxid-Verbindungen bilden, die anschließend, d.h. in einer zweiten Stufe, im Oxidationsreaktor für die Oxidation von aromatischen Kohlenwasserstoffen zu Carbonsäureanhydriden und/oder Carbonsäuren in situ unter den Bedingungen dieser Oxidation in die gewünschten, katalytisch aktiven Silber-Vanadiumoxid-Bronzen umgewandelt werden. Es kann auch z. B. der Katalysatorträger unter für die Bildung der Silber-Vanadiumoxid-Bronze nicht optimierten Bedingungen bei Temperaturen von oberhalb 200°C bis 500°C mit dem erfindungsgemäßen Multimetalloxid beschichtet werden, so daß beim Beschichtungsvorgang aus dem Multimetalloxid nicht näher definierbare Silber-Vanadiumoxid-Verbindungen entstehen, und der so beschichtete Träger, gegebenenfalls nach einer weiteren thermischen Behandlung bei oberhalb 200 bis 650°C im Oxidationsreaktor zur Oxidation von aromatischen Kohlenwasserstoffen zu Carbonsäureanhydriden und/oder Carbonsäuren unter den Bedingungen dieser Oxidation in situ in einen erfindungsgemäßen Schalenkatalysator umgewandelt wird.

Eine andere Möglichkeit zur Herstellung eines erfindungsgemäßen Schalenkatalysators besteht in der thermischen Behandlung des erfindungsgemäßen Multimetalloxidpulvers bei Temperaturen von oberhalb 200°C bis 650°C und der Beschichtung des inerten, nicht-porösen Katalysatorträgers, gegebenenfalls unter Zusatz eines Bindemittels, mit der hierbei gegebenenfalls erhaltenen Silber-Vanadiumoxid-Bronze oder den hierbei gegebenenfalls erhaltenen, vorgenannten kristallographisch nicht aufgeklärten Silber-Vanadiumoxid-Verbindungen. Im Falle der Beschichtung des Katalysator-trägers mit der erhaltenen Silber-Vanadiumoxid-Bronze entsteht hierbei ein erfindungsgemäßer Schalenkatalysator; im Falle der Beschichtung des Katalysatorträgers mit den gegebenenfalls erhaltenen, vorgenannten kristallographisch nicht aufgeklärten Silber-Vanadiumoxid-Verbindungen wird der beschichtete Katalysatorträger vorzugsweise unter den Bedingungen der Oxidation von aromatischen Kohlenwasserstoffen zu Carbonsäureanhydriden und/oder Carbonsäuren in situ im Oxidationsreaktor in einen erfindungsgemäßen Schalenkatalysator umgewandelt.

Besonders bevorzugt werden die erfindungsgemäßen Schalenkatalysatoren allerdings aus den erfindungsgemäßen Präkatalysatoren einstufig oder gegebenenfalls, nach einer thermischen Behandlung im Zuge oder nach der Beschichtung des Katalysatorträgers, mehrstufig, insbesondere einstufig, jeweils in situ im Oxidationsreaktor unter den Bedingungen der Oxidation von aromatischen Kohlenwasserstoffen zu Carbonsäureanhydriden und/oder Carbonsäuren, erzeugt.

Die katalytisch aktive Schale des erfindungsgemäß hergestellten Schalenkatalysators enthält im allgemeinen 30 bis 100 Gew.-%, vorzugsweise 50 bis 100 Gew.-%, bezogen auf das Gesamtgewicht der katalytisch aktiven Schale, der so erzeugten Silber-Vanadiumoxid-Bronzen, wobei das Silber und das Vanadium in der katalytisch aktiven Schale im allgemeinen in einem Atomverhältnis Ag : V von 0,15 bis 0,95, vorzugsweise von 0,25 bis 0,5 und besonders bevorzugt von 0,3 bis 0,45 vorliegen. Besonders bevorzugt besteht die katalytisch aktive Schicht der erfindungsgemäßen Schalenkatalysatoren vollständig aus den erfindungsgemäß erzeugten Silber-Vanadiumoxid-Bronzen. Enthält die katalytisch aktive Schicht oder Schichten außer den erfindungsgemäß erzeugten Silber-Vanadiumoxid-Bronzen noch weitere Komponenten, können dies z.B. Inertmaterialien des Standes der Technik, wie Siliciumcarbid oder Steatit, sein oder aber auch nicht-erfindungsgemäße Katalysatorverbindungen zur Oxidation von aromatischen Kohlenwasserstoffen zu Carbonsäuren und/oder Carbonsäureanhydriden z. B. auf Vanadiumpentoxid/Anatas-Basis, wie sie eingangs beispielhaft bei der Schilderung des Standes der Technik erwähnt wurden. Die Schichtdicke der die katalytisch aktiven Bestandteile enthaltenden Katalysatorschale beträgt im allgemeinen 10 bis 250 µm. Dies gilt auch, falls die Katalysatorschale aus mehreren, nacheinander aufgetragenen Schichten besteht.

Überraschenderweise haben die erfindungsgemäßen Schalenkatalysatoren gegenüber Katalysatoren des Standes der Technik auf Silber-Vanadiumoxid-Basis (z.B. E.I. Andreikov; V. Volkov; Kinet. Katal. 22, 963 (1981) und Kinet. Katal. 22, 1207 (1981)) trotz ähnlicher Röntgenbeugungsdiagramme verbesserte Eigenschaften bei der Oxidation von aromatischen Kohlenwasserstoffen zu Carbonsäuren und/oder Carbonsäureanhydriden. Dies ist vermutlich auf die im Vergleich zu dem genannten Stand der Technik höhere BET-Oberfläche der erfindungsgemäßen Schalenkatalysatoren zurückzuführen, die im allgemeinen 2 bis 100 m²/g vorzugsweise 2 bis 40 m²/g und besonders bevorzugt 3 bis 20 m²/g beträgt und somit um ein Vielfaches höher ist, als nach dem Stand der Technik erzielbar. Offensichtlich führt die Verwendung der erfindungsgemäßen Multimetalloxide zur Herstellung des Schalenkatalysators, vorzugsweise über die Stufe des Präkatalysators, zu einer größeren BET-Oberfläche der daraus erzeugten katalytisch aktiven Silber-Vanadiumoxid-Bronzen.

Die erfindungsgemäßen Schalenkatalysatoren werden für die partielle Oxidation von aromatischen Kohlenwasserstoffen zu Carbonsäuren und/oder Carbonsäureanhydriden, insbesondere zur Gasphasenpartialoxidation von o-Xylol und/oder Naphthalin zu Phthalsäureanhydrid oder von Toluol zu Benzoesäure und Benzaldehyd, mit einem molekularen Sauerstoff enthaltenden Gas verwendet. Die erfindungsgemäßen Katalysatoren können zu diesem Zweck alleine oder in Kombination mit anderen, unterschiedlich aktiven Katalysatoren, beispielsweise Katalysatoren des Standes der Technik auf Vanadiumoxid/Anatas-Basis, eingesetzt werden, wobei die unterschiedlichen Katalysatoren im allgemeinen in separaten Katalysatorschüttungen, die in einem oder mehreren Katalysatorfestbetten angeordnet sein können, im Reaktor angeordnet werden.

Die erfindungsgemäßen Schalenkatalysatoren oder Präkatalysatoren werden hierzu in die Reaktionsrohre eines Röhrenreaktors gefüllt, die von außen, z.B. mittels einer Salzschmelze, auf die Reaktionstemperatur thermostatisiert werden. Wird anstelle des erfindungsgemäßen Schalenkatalysators ein erfindungsgemäßer Präkatalysator eingesetzt, entsteht daraus unter den Temperaturbedingungen der partiellen Oxidation von aromatischen Kohlenwasserstoffen zu Carbonsäuren und/oder Carbonsäureanhydriden, insbesondere bei der Partialoxidation von o-Xylol und/oder Naphthalin zu PSA oder bei der Partialoxidation von Toluol zu Benzoesäure und Benzaldehyd, ein erfindungsgemäßer Schalenkatalysator. Über die so bereitete Katalysatorschüttung wird das Reaktionsgas bei Temperaturen von im allgemeinen 300 bis 450°C, vorzugsweise 320 bis 420°C und besonders bevorzugt von 340 bis 400°C und bei einem Überdruck von im allgemeinen 0,1 bis 2,5 bar, vorzugsweise von 0,3 bis 1,5 bar mit einer Raumgeschwindigkeit von im allgemeinen 750 bis 5000 h⁻¹ geleitet.

Das dem Katalysator zugeführte Reaktionsgas wird im allgemeinen durch Vermischen von einem molekularen Sauerstoff enthaltenden Gas, daß außer Sauerstoff noch geeignete Reaktionsmoderatoren und/oder Verdünnungsmittel, wie Wasserdampf, Kohlendioxid und/oder Stickstoff, enthalten kann, mit dem zu oxidierenden, aromatischen Kohlenwasserstoff erzeugt, wobei das molekularen Sauerstoff enthaltende Gas im allgemeinen 1 bis 100 Vol.-%, vorzugsweise 2 bis 50 Vol.-% und besonders bevorzugt 10 bis 30 Vol.-% Sauerstoff, 0 bis 30 Vol.-%, vorzugsweise 0 bis 10 Vol.-% Wasserdampf sowie 0 bis 50 Vol.-%, vorzugsweise 0 bis 1 Vol.-% Kohlendioxid, Rest Stickstoff, enthalten kann. Zur Erzeugung des Reaktionsgases wird das molekularen Sauerstoff enthaltende Gas im allgemeinen mit 30 bis 300 g je Nm³, bevorzugt mit 70 bis 150 g je Nm³ Gas des zu oxidierenden aromatischen Kohlenwasserstoffs beschickt. Besonders vorteilhaft wird als molekularen Sauerstoff enthaltendes Gas Luft verwendet

Vorteilhaft wird die Gasphasenpartialoxidation so durchgeführt, daß man zwei oder mehr Zonen, vorzugsweise zwei Zonen, der im Reaktionsrohr befindlichen Katalysatorschüttung auf unterschiedliche Reaktionstemperaturen thermostatisiert, wozu beispielsweise Reaktoren mit getrennten Salzbädern, wie sie in DE-A 22 01 528 oder DE-A 28 30 765 beschrieben sind, eingesetzt werden können. Wird die Umsetzung in zwei Reaktionszonen durchgeführt, wie in DE-A 40 13 051 beschrieben, wird im allgemeinen die zum Gaseintritt des Reaktionsgases hin gelegene Reaktionszone, welche im allgemeinen 30 bis 80 Vol.-% des gesamten Katalysatorvolumens umfaßt, auf eine um 1 bis 20°C, vorzugsweise um 1 bis 10°C und insbesondere um 2 bis 8°C höhere Reaktionstemperatur als die zum Gasaustritt hin gelegene Reaktionszone thermostatisiert. Eine solche Arbeitsweise wird als Zwei- oder Mehrzonenstrukturierung des Reaktors bezeichnet. Alternativ kann die Gasphasenoxidation auch ohne Aufteilung in Temperaturzonen bei einer einheitlichen Reaktionstemperatur durchgeführt werden.

Bei einer bevorzugten Ausführungsform des Verfahrens zur partiellen Oxidation von aromatischen Kohlenwasserstoffen zu Carbonsäuren und/oder Carbonsäureanhydriden, die sich besonders vorteilhaft für die Herstellung von Phthalsäureanhydrid aus o-Xylol und/oder Naphthalin erweist, wird der aromatisch Kohlenwasserstoff, z.B. o-Xylol, zunächst an einer Schüttung des erfindungsgemäßen Schalenkatalysators unter Teilumsatz zu Phthalsäureanhydrid und anderen Oxidationsprodukten, wie o-Tolylaldehyd, o-Tolylcarbonsäure und Phthalid, umgesetzt. Das resultierende Produktgemisch, das zusätzlich nicht umgesetztes o-Xylol enthält, kann dann weiterverarbeitet werden, indem, alternativ, entweder
a) das o-Xylol vom Phthalsäureanhydrid und den anderen obengenannten Oxidationsprodukten, die Intermediate auf dem Reaktionsweg von o-Xylol zu Phthalsäureanhydrid sind, abgetrennt und zurückgeführt wird und der Strom aus Phthalsäureanhydrid und Intermediaten einer oder mehreren weiteren Katalysatorschüttungen mit z. B. einem Schalenkatalysator auf Vanadiumoxid/Anatas-Basis zugeführt wird, wo die Intermediate selektiv zu Phthalsäureanhydrid oxidiert werden; oder indem
b) das Produktgemisch ohne weitere Aufarbeitung, d.h. ohne o-Xylol-Abtrennung, über eine zweite oder gegebenenfalls über weitere Katalysatorschüttungen geleitet wird, wie sie gemäß Stand der Technik zur Herstellung von Phthalsäureanhydrid aus o-Xylol verwendet werden können, z.B. Schalenkatalysatoren auf Vanadiumoxid/Anatas-Basis als katalytisch-aktiven Bestandteilen. Dies kann unter Anwendung einer Zwei- oder Mehrzonenstrukturierung im selben Reaktor geschehen oder auch unter Anwendung eines Nachreaktors.

Durch diese Art der Reaktionsführung wird insgesamt eine deutlich höhere Phthalsäureanhydrid-Ausbeute erzielt als mit Katalysatoren des Standes der Technik allein, da die erfindungsgemäßen Schalenkatalysatoren, o-Xylol und/oder Naphthalin wesentlich selektiver zu Phthalsäureanhydrid bzw. den vorstehend genannten Intermediaten oxidieren können, als dies bei alleiniger Verwendung von Katalysatorsystemen auf Vanadiumoxid/Anatas-Basis gemäß Stand der Technik möglich ist. Durch die vorstehend genannte Kombination von Katalysatorschüttungen mit dem erfindungsgemäßen Schalenkatalysator in der ersten Reaktionszone und einer oder mehrerer aus Schalenkatalysatoren auf Vanadiumoxid/Anatas-Basis wird darüber hinaus der vollständige Umsatz des eingesetzten o-Xylols bei einer gleichzeitig hohen Selektivität für die Bildung von Phthalsäureanhydrid mit einer hohen Produktqualität ermöglicht.

Auf analoge Weise kann bei der Oxidation von Toluol zu Benzoesäure verfahren werden, wobei zunächst ein Gemisch aus nicht umgesetztem Toluol, Benzoesäure und Benzaldehyd entsteht. Alternativ kann gewünschtenfalls auch das Nebenprodukt Benzaldehyd isoliert werden, der ebenfalls ein Wertprodukt darstellt und beispielsweise als Aromastoff Verwendung findet.

### Beispiel 1 (Herstellung von HNO₃-haltigem Ag_{0,73}V₂Oₓ)

In 7 1 vollentsalztes Wasser von 60°C wurden 90,95 g V₂O₅ (= 0,5 Mol) unter Rühren zugegeben. In die erhaltene orange-farbene Suspension wurde unter weiterem Rühren eine wässrige Lösung von 62,0 g AgNO₃ (=0,365 Mol) in 1 1 Wasser zugegeben. Anschließend wurde die Temperatur der erhaltenen Suspension innerhalb von 2 Stunden auf 90°C erhöht und bei dieser Temperatur die Mischung 24 Stunden gerührt. Danach wurde die erhaltene dunkelbraune Suspension abgekühlt und sprühgetrocknet (Eingangstemperatur (Luft) = 380°C, Ausgangstemperatur (Luft) = 104°C).

Das erhaltene Pulver hatte eine spezifische Oberfläche nach BET von 45,0 m²/g. Die chemische Analyse ergab ein Ag/V-Atomverhältnis von 0,38. Vom erhaltenen Pulver wurde ein Pulverröntgendiagramm mit Hilfe eines Siemens Diffraktometers D 5000 unter Anwendung von Cu-K_{α}-Strahlung (40 kV, 30 mA) aufgenommen. Das Diffraktometer war mit einem automatischen Primär- und Sekundärblendensystem sowie einem Sekundär-Monochromator und Szintillations-Detektor ausgestattet. Tabelle 3 zeigt das am erhaltenen Pulver im 2θ-Bereich von 5 bis 65° gemessene Pulverröntgendiagramm, wiedergegeben in Gestalt der von der Wellenlänge der verwendeten Röntgenstrahlung unabhängigen Netzebenenabstände d [Å] sowie die zugehörigen, auf den intensitätstärksten Beugungsreflex bezogenen, relativen Intensitäten Iᵣₑₗ[%] der verschiedenen Beugungsreflexe. Die relativen Intensitäten wurden aus den Peakhöhen der Beugungsreflexe ermittelt.

**Tabelle 3:**

| d [Å] | Iᵣₑₗ [%] |
|---|---|
| 15,23 | 16 |
| 12,16 | 11 |
| 10,68 | 18 |
| 7,16 | 6 |
| 6,10 | 5 |
| 5,24 | 5 |
| 5,06 | 11 |
| 4,37 | 23 |
| 4,12 | 7 |
| 4,02 | 8 |
| 3,86 | 16 |
| 3,51 | 14 |
| 3,41 | 80 |
| 3,26 | 13 |
| 3,09 | 61 |
| 3,02 | 100 |
| 2,78 | 13 |
| 2,71 | 10 |
| 2,58 | 23 |
| 2,50 | 21 |
| 2,48 | 24 |
| 2,42 | 23 |
| 2,36 | 38 |
| 2,30 | 17 |
| 2,25 | 14 |
| 2,10 | 13 |
| 2,04 | 26 |
| 1,93 | 31 |
| 1,85 | 13 |
| 1,80 | 43 |
| 1,76 | 19 |
| 1,70 | 18 |
| 1,55 | 36 |
| 1,53 | 33 |
| 1,49 | 17 |
| 1,44 | 14 |

In der folgenden Tabelle 4 sind zum Vergleich die entsprechenden Literaturdaten für β-Ag_{0,35}V₂O₅ und δ-Ag_{0,8}V₂O₅ aufgeführt (aus: A. Casalot, M. Pouchard: Bull. Soc. Chim. France 3817 (1967); Tableau III)

**Tabelle 4:**

| β-Ag_{0,35}V₂O₅ | | δ-Ag_{0,80}V₂O₅ | |
|---|---|---|---|
| d (Å) | I/Iₒ | d (Å) | I/Iₒ |
| 7,20 | 12 | 4,85 | 20 |
| 6,96 | 8 | 4,38 | 2 |
| 4,72 | 40 | 3,507 | 24 |
| 3,83 | 40 | 3,232 | 72 |
| 3,497 | 12 | 2,910 | 100 |
| 3,367 | 20 | 2,768 | 40 |
| 3,045 | 100 | 2,544 | 32 |
| 2,910 | 55 | 2,418 | 16 |
| 2,887 | 50 | 2,270 | 2 |
| 2,720 | 38 | 2,241 | 2 |
| 2,616 | 16 | 2,189 | 4 |
| 2,443 | 12 | 1,967 | 4 |
| 2,363 | 12 | 1,945 | 8 |
| 2,164 | 17 | 1,916 | 3 |
| 1,971 | 25 | 1,855 | 16 |
| 1,861 | 12 | 1,828 | 24 |
| 1,802 | 30 | 1,754 | 6 |

### Beispiel 2 (Herstellung von Nitrat-freiem Ag_{0,73}V₂Oₓ)

Die gemäß Beispiel 1 erhaltene dunkelbraune Suspension wurde über ein Filter abgesaugt und mit 7 1 Wasser nachgewaschen. Das zuletzt erhaltene Filtrat war praktisch Silber-frei. Der erhaltene dunkelbraune Filterkuchen wurde bei 110°C im Vakuumtrockenschrank 15 Stunden lang getrocknet.

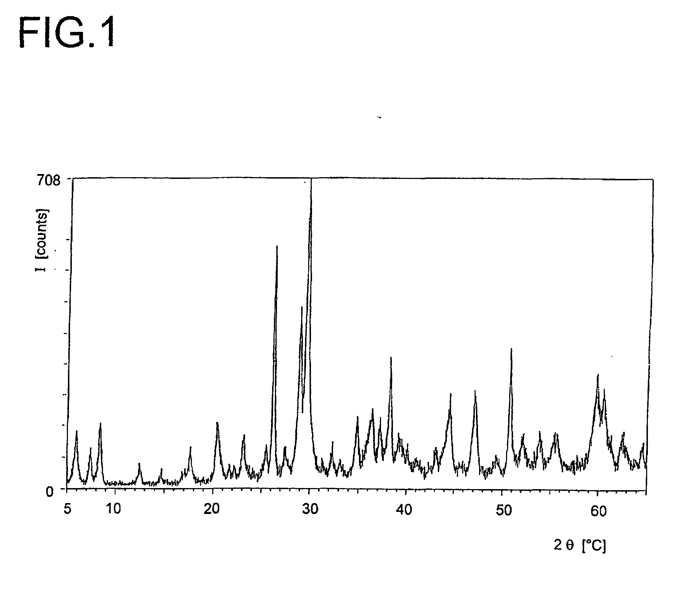

Das erhaltene Pulver wies eine spezifische Oberfläche nach BET von 47,5 m²/g auf. Die chemische Analyse ergab ein Ag/V-Atomverhältnis von 0,34. Eine potentiometrische Bestimmung des Oxidationszustands der Vanadiumkomponente im erhaltenen Pulver zeigte das Vorliegen von ganz überwiegend Vanadium(V) (37,7 Gew.-%) neben sehr wenig Vanadium(IV) (0,2 Gew.-%). Wie die rasterelektronenmikroskopische Untersuchung zeigt, weist das erhaltene Pulver eine faserförmige Morphologie auf. Das Pulverröntgenbeugungsdiagramm stimmte mit dem des Produkts des Beispiels 1 überein. Das Pulverröntgenbeugungsdiagramm ist in Fig. 1 abgebildet.

### Beispiel 3 (Herstellung der Vergleichskatalysatoren)

### Vergleichskatalysator (a)

50,0 kg Ringe aus Steatit (Magnesiumsilikat) mit einem äußeren Durchmesser von 8 mm, einer Länge von 6 mm und einer Wandstärke von 1,5 mm wurden in einer Dragiertrommel auf 160°C erhitzt und mit einer Suspension von 25,0 kg Anatas einer BET-Oberfläche von 20 m²/g, 1,81 kg Vanadyloxalat, 0,143 kg Cäsiumsulfat, 38 kg Wasser und 9,85 kg Formamid solange besprüht, bis das Gewicht der auf diese Weise aufgetragenen Schicht 10,0 % des Gesamtgewichts (nach Calcination bei 450°C; für diese Bestimmung werden zu verschiedenen Zeitpunkten Proben aus der Dragiertrommel entnommen und bei 450°C calciniert) des fertigen Schalenkatalysators betrug. Die auf diese Weise aufgebrachte katalytisch aktive Masse, also die Katalysatorschale, bestand aus 0,40 Gew.-% Cäsium (berechnet als Cs), 4,0 Gew.-% Vanadium (berechnet als V₂O₅) und 95,6 Gew.-% Titandioxid (ber. als TiO₂).

### Vergleichskatalysator (b)

50 kg Ringe aus Steatit (Magnesiumsilikat) mit einem äußeren Durchmesser von 8 mm, einer Länge von 6 mm und einer Wandstärke 5 von 1,5 mm wurden in einer Dragiertrommel auf 160°C erhitzt und mit einer Suspension aus 28,6 kg Anatas mit einer BET-Oberfläche von 20 m²/g, 4,11 kg Vanadyloxalat, 1,03 kg Antimontrioxid, 0,179 kg Ammoniumdihydrogenphosphat, 0,046 kg Cäsiumsulfat, 44,1 kg Wasser und 9,14 kg Formamid besprüht, bis das Gewicht der aufgetragenen Schicht 10,5 % des Gesamtgewichts des fertigen Katalysators betrug (nach Calcination bei 450°C). Die auf diese Weise aufgebrachte katalytisch aktive Masse, also die Katalysatorschale, bestand aus 0,15 Gew.-% Phosphor (berechnet als P), 7,5 Gew.-% Vanadium (berechnet als V₂O₅), 3,2 Gew.-% Antimon (berechnet als Sb₂O₃), 0,1 Gew.-% Cäsium (berechnet als Cs) und 89,05 Gew.-% Titandioxid (berechnet als TiO₂).

### Erfindungsgemäßer Katalysator (c)

### ("Präkatalysator")

Das nach Beispiel 1 hergestellte HNO₃-haltige Ag_{0,73}V₂Oₓ-Pulver wurde, wie folgt, auf Magnesiumsilikat-Ringe aufgebracht: 700 g Steatit-Ringe mit einem äußeren Durchmesser von 8 mm, einer Länge von 6 mm und einer Wandstärke von 1,5 mm wurden in einer Dragiertrommel bei 20°C während 20 Minuten mit 115 g des HNO₃-haltigen Ag_{0,73}V₂Oₓ-Pulvers unter Zusatz von 56 g eines 30 Gew.-% Wasser und 70 Gew.-% Glycerin enthaltenden Gemisches beschichtet und anschließend getrocknet. Das Gewicht der so aufgetragenen katalytisch aktiven Masse betrug nach Wärmebehandlung bei 400°C für 1/2 h 12,9 Gew.-%, bezogen auf das Gesamtgewicht des fertigen Katalysators. Diese Gewichtsbestimmung wurde jeweils mit zu verschiedenen Zeitpunkten der Dragiertrommel entnommenen Probemengen des Präkatalysators durchgeführt; der Präkatalysator selbst wurde bei seiner Herstellung nicht auf 400°C erhitzt.

### Beispiel 4 (Herstellung von Phthalsäureanhydrid mit den Vergleichskatalysatoren 3 (a) und 3 (b))

Von unten nach oben wurden jeweils 1,30 m des Katalysators 3 b und anschließend 1,60 m des Katalysators 3 a in ein 3,85 m langes Eisenrohr mit einer lichten Weite von 25 mm eingefüllt. Das Eisenrohr war zur Temperaturregelung von einer Salzschmelze umgeben. Durch das Rohr wurden stündlich von oben nach unten 4,0 Nm³-Luft geleitet. Dabei wurde bei Beladungen mit 98,5 gew.-%igem o-Xylol von 60-80 g o-xylol/Nm³ Luft und einer Salzbadtemperatur von 352-355°C eine durchschnittliche Phthalsäureanhydrid-(PSA)-Ausbeute von 113,3 Gew.-% erreicht (Ausbeute bedeutet das erhaltene PSA in Gewichtsprozent, bezogen auf 100%iges o-Xylol). Der Umsatz betrug >99,95 %, der Rest-Phthalid-Gehalt am Reaktorausgang lag bei <0,20 Gew.-%.

Beispiel 5 (Herstellung von Phthalsäureanhydrid mit einer Kombination des erfindungsgemäßen Präkatalysators 3(c) mit den bekannten Katalysatoren 3(a) und 3(b) in einem Rohr).

Von unten nach oben wurden jeweils 0,90 m des Katalysators 3(b), 0,80 m des Katalysators 3(a) und anschließend 1,20 m des Präkatalysators 3(c) in ein 3,85 m langes Eisenrohr mit einer lichten Weite von 25 mm eingefüllt. Das Eisenrohr war zur Temperaturregelung von einer Salzschmelze umgeben. Durch das Rohr wurden stündlich von oben nach unten 4,0 Nm³ Luft mit Beladungen an 98,5 gew.-%igem o-Xylol von 60 bis 80 g o-Xylol/Nm³-Luft geleitet. Dabei wurde bei 60-80 g Beladung und einer Salzbadtemperatur von 353-358°C eine durchschnittliche PSA-Ausbeute von 115,4 Gew.-% erreicht (Ausbeute bedeutet das erhaltene Phthalsäureanhydrid in Gewichtsprozent, bezogen auf 100%iges o-Xylol). Der Umsatz betrug > 99,94 %, der Rest-Phthalid-Gehalt am Reaktorausgang lag bei < 0,20 Gew.-%.

### Beispiel 6 (Vergleich mit Ag-V₂O₅-Bronze bei Teilumsatz)

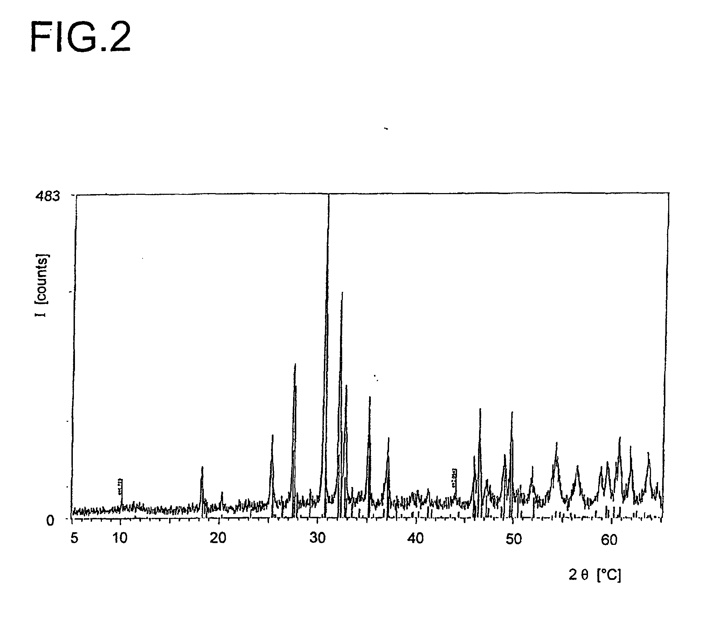

a) Herstellung des Vergleichskatalysators 6a
   Eine Mischung aus 90,95 g V₂O₅ (0,5 Mol) und 62,0 g AgNO₃ (0,365 Mol) wurde in Anlehnung an die Angaben der Literaturstelle (E.I. Andreikov, V.L. Volkov, Kin. Katal. 22, 963 (1981)) durch thermische Behandlung bei 750°C an der Luft umgesetzt. Es bildete sich eine Schmelze der Brutto-Zusammensetzung Ag_{0,73}V₂Oₓ. Die erstarrte Schmelze wurde zu einem Pulver mit einer Korngrößenverteilung von 1-10 µm gemahlen. Eine Röntgenbeugungsaufnahme dieses Pulvers ergab, daß das Vergleichs-Ag-V-Oxid aus Ag_{1,2}V₃O₈ (Hauptprodukt) und β-Ag-V₂O₅-Bronze (Nebenprodukt) bestand. Die für die erfindungsgemäßen Multimetalloxide charakteristischen Beugungslinien bei d = 15,23 + 0,6, 12,16 + 0,4, 10,68 + 0,3, 3,41 ± 0,04 , 3,09 ± 0,04 , 3,02 ± 0,04 , 2,36 ± 0,04 und 1,80 ± 0,04 Å (wiedergegeben in Form der von der Wellenlänge der verwendeten Röntgenstrahlung unabhängigen Netzebenenabstände d [Å]) wurden nicht gefunden. Das so hergestellte Pulver wurde, wie folgt, auf Magnesiumsilikat-Kugeln aufgebracht: 700 g Steatit-Kugeln mit einem Durchmesser von 3,5 mm wurden in einer Dragiertrommel bei 20°C während 20 Minuten mit 123,9 g des Ag_{0,73}V₂Oₓ-Pulvers unter Zusatz von 45 g eines 70 Gew.-% Wasser und 30 Gew.-% Glycerin enthaltenden Gemisches beschichtet. Das Gewicht der so aufgetragenen katalytisch aktiven Masse betrug nach Wärmebehandlung für 1/2 h bei 400°C 15,0 Gew.-%, bezogen auf das Gesamtgewicht des fertigen Katalysators.
b) Herstellung des erfindungsgemäßen Katalysators 6b
   Das nach dem Beispiel 1 hergestellte HNO₃-haltige Ag_{0,73}V₂Oₓ-Pulver wurde, wie folgt, auf Magnesiumsilikat-Kugeln aufgebracht: 700 g Steatit-Kugeln mit einem Durchmesser von 3,5 mm wurden in einer Dragiertrommel bei 20°C während 20 min mit 135,9 g des Pulvers aus Beispiel 1 unte Zusatz von 62 g eines 70 Gew.-%-Wasser und 30 Gew.-% Glycerin enthaltenden Gemisches beschichtet und anschließend getrocknet. Das Gewicht der so aufgetragenen katalytisch aktiven Masse, bestimmt an einer Probe des erhaltenen Präkatalysators, betrug nach Wärmebehandlung bei 400°C für 1/2 h 14,9 Gew.-%, bezogen auf das Gesamtgewicht des fertigen Katalysators.
c) Herstellung von Phthalsäureanhydrid mit dem Vergleichskatalysator 6a sowie mit dem erfindungsgemäßen Katalysator 6b
   In ein 80 cm langes Eisenrohr mit einer lichten Weite von 15 mm wurden jeweils 135 g des Katalysators 6a bzw. des Katalysators 6b eingefüllt. Das Eisenrohr war zur Temperaturregelung von einer Salzschmelze umgeben. Durch das Rohr wurden stündlich von oben nach unten 360 Nl-Luft mit Beladungen an 98,5 gew.-%igem o-Xylol von 40-50 g o-Xylol/Nm³ Luft geleitet. Nach beendeter Reaktion wurden die Katalysatorausbauproben bezüglich ihrer Phasenzusammensetzung und BET-Oberfläche untersucht. Diese Untersuchungen wurden an einer Abriebprobe der Schale des aus dem Reaktor nach Beendigung der Umsetzung ausgebauten Katalysators durchgeführt. Die BET-Oberfläche wurde nach DIN 66 131 bestimmt, die Phasenzusammensetzung mittels Röntgenstrukturanalyse. Fig. 2 ist die Abbildung des erhaltenen Pulverröntgenbeugungsdiagramms dieser Ausbauprobe. Der Vergleich der Röntgenbeugungsdiagramme in Fig. 1 und Fig. 2 belegt die Umwandlung der erfindungsgemäßen Multimetalloxide zu einem Gemisch aus Silber-Vanadiumoxid-Bronzen unter den Bedingungen der PSA-Herstellung. Der Vergleich der Röntgenbeugungsdiagramme von Fig. 1 und Fig. 2 belegt weiterhin, daß die erfindungsgemäßen Multimetalloxide eine neue Phase, d.h. eine neue Verbindung sind und nicht aus einem Gemisch aus Silber-Vanadiumoxid-Bronzen bestehen. In der nachstehenden Tabelle 5 sind die erhaltenen Ergebnisse zusammengefaßt.

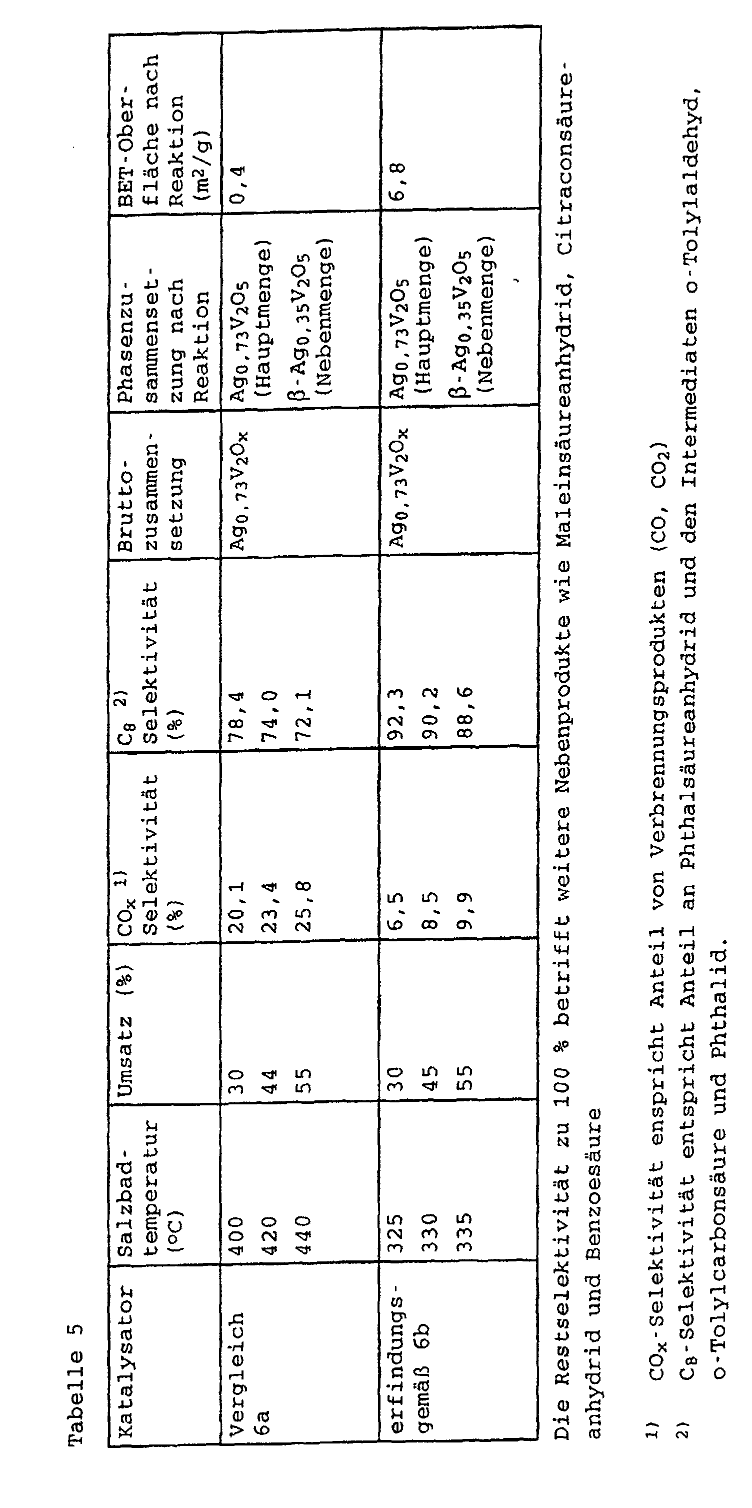

### Beispiel 7

### Herstellung von Benzoesäure/Benzaldehyd mit dem Vergleichskatalysator 6a sowie mit dem erfinderischen Katalysator 6b

In ein 80 cm langes Eisenrohr mit einer lichten Weite von 15 mm wurden jeweils 135 g des Katalysators 6a bzw. 6b eingefüllt. Das Eisenrohr war zur Temperaturregelung von einer Salzschmelze umgeben. Durch das Rohr wurde stündlich von oben nach unten ein Gasgemisch, bestehend aus 360 Nl-Luft und 30-40 Nl-Wasserdampf mit Beladungen an 99,5 gew.-%igem Toluol von 40-50 g Toluol/Nm³-Luft, geleitet. Nach beendeter Reaktion wurden die Katalysatorausbauproben bezüglich ihrer Phasenzusammensetzung und BET-Oberfläche, wie in Beispiel 6c) beschrieben, untersucht. Tabelle 6 faßt die erhaltenen Ergebnisse zusammen.

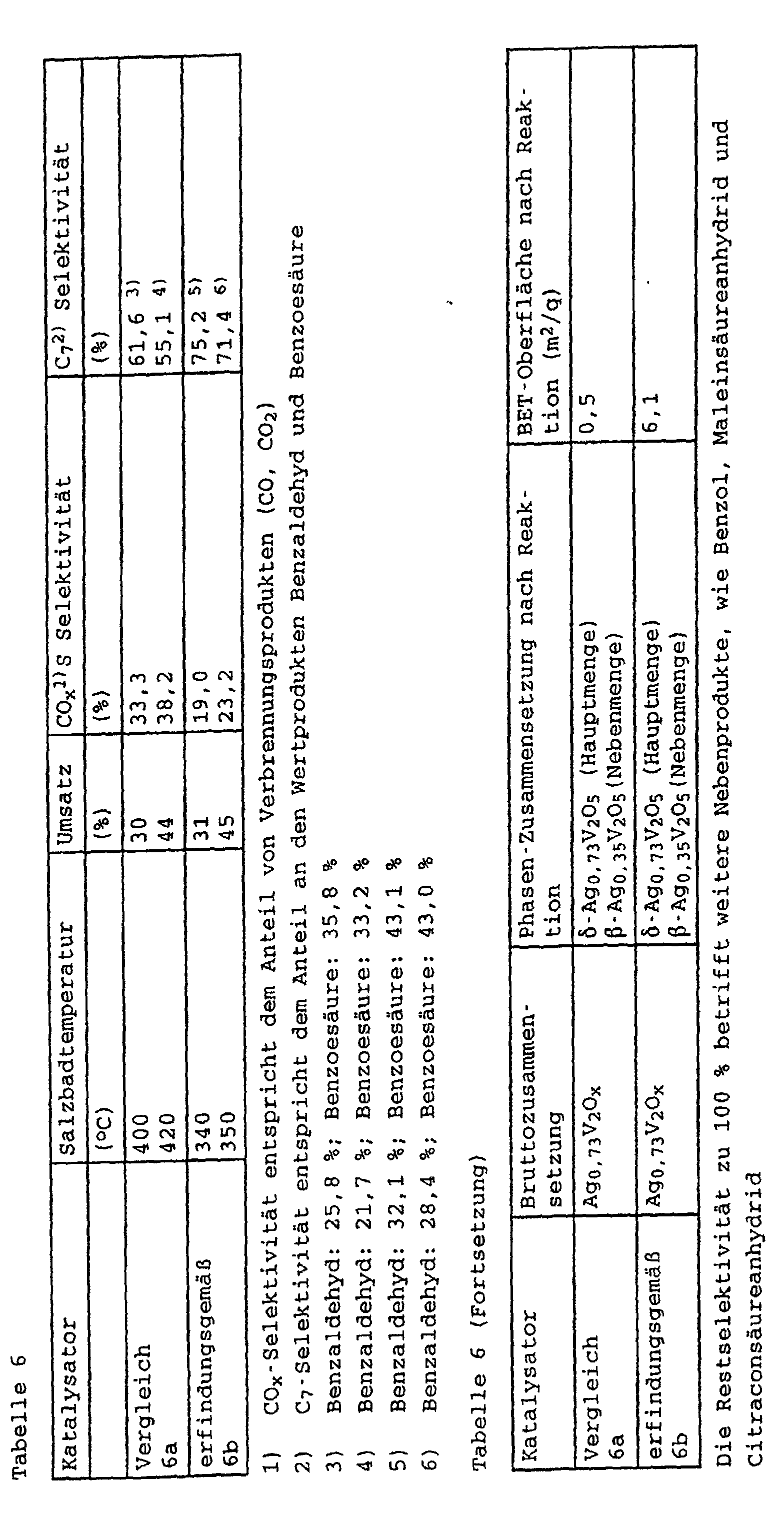

## Patentansprüche

1. Multimetalloxid der allgemeinen Formel I
Ag_{a-b}M_{b}V₂Oₓ * c H₂O, I
in der M ein Metall ausgewählt aus der Gruppe Li, Na, K, Rb, Cs, Tl, Mg, Ca, Sr, Ba, Cu, Zn, Cd, Pb, Cr, Au, Al, Fe, Co, Ni und/oder Mo ist,
a einen Wert von 0,3 bis 1,9 und
b einen Wert von 0 bis 0,5 hat, mit der Maßgabe, daß die Differenz (a-b) ≥ 0,1 ist und
c einen Wert von 0 bis 20 hat und
x eine Zahl, die sich durch die Wertigkeit und Häufigkeit der von Sauerstoff verschiedenen Elemente in der Formel I bestimmt, bedeutet,
das in einer Kristallstruktur vorliegt, die ein Pulverröntgendiagramm ergibt, welches Beugungsreflexe bei den Netzebenenabständen d 15,23 ± 0,6, 12,16 ± 0,4, 10,68 ± 0,3, 3,41 ± 0,04, 3,09 ± 0,04, 3,02 ± 0,04, 2,36 ± 0,04 und 1,80 ± 0,04 Å hat.

2. Multimetalloxid gemäß Anspruch 1, das eine faserförmige Kristallmorphologie mit einem mittleren Verhältnis aus Faserdurchmesser zu Faserlänge von kleiner 0,6 aufweist.

3. Multimetalloxid gemäß Anspruch 1, das eine spezifische Oberfläche nach BET von 3 bis 250 m²/g hat.

4. Multimetalloxid gemäß Anspruch 1, in dem a einen Wert von 0,5 bis 1,0, b einen Wert von 0 bis 0,3 und c einen Wert von 0 bis 5 hat.

5. Multimetalloxid gemäß Anspruch 1, in dem a einen Wert von 0,6 bis 0,9 hat, b einen Wert von 0 bis 0,1 hat und c einen Wert von 0 bis 1 hat.

6. Multimetalloxid gemäß Anspruch 1 der Formel
AgₐV₂Oₓ* c H₂O,
in der a einen Wert von 0,6 bis 0,9 hat, x die in Anspruch 1 genannte Bedeutung hat und c einen Wert von 0 bis 5 hat.

7. Multimetalloxid gemäß Anspruch 1, dessen Pulverröntgendiagramm die folgenden 17 Beugungsreflexe bei den Netzebenenabständen d [Å] aufweist:
| Beugungsreflexe | d [Å] |
|---|---|
| 1 | 15,23 ± 0,6 |
| 2 | 12,16 ± 0,4 |
| 3 | 10,68 ± 0,3 |
| 4 | 5,06 ± 0,06 |
| 5 | 4,37 ± 0,04 |
| 6 | 3,86 ± 0,04 |
| 7 | 3,41 ± 0,04 |
| 8 | 3,09 ± 0,04 |
| 9 | 3,02 ± 0,04 |
| 10 | 2,58 ± 0,04 |
| 11 | 2,48 ± 0,04 |
| 12 | 2,42 ± 0,04 |
| 13 | 2,36 ± 0,04 |
| 14 | 2,04 ± 0,04 |
| 15 | 1,93 ± 0,04 |
| 16 | 1,80 ± 0,04 |
| 17 | 1,55 ± 0,04 |

8. Multimetalloxid gemäß Anspruch 7, dessen Beugungsreflexe 1 bis 17 die nachfolgenden ungefähren relativen Intensitäten (Iᵣₑₗ [%]) aufweisen:
| Beugungsreflexe | Iᵣₑₗ[%] |
|---|---|
| 1 | 16 |
| 2 | 11 |
| 3 | 18 |
| 4 | 11 |
| 5 | 23 |
| 6 | 16 |
| 7 | 80 |
| 8 | 61 |
| 9 | 100 |
| 10 | 23 |
| 11 | 24 |
| 12 | 23 |
| 13 | 38 |
| 14 | 26 |
| 15 | 31 |
| 16 | 43 |
| 17 | 36 |

9. Verfahren zur Herstellung von Multimetalloxiden gemäß Anspruch 1, **dadurch gekennzeichnet, daß** man in einer Flüssigkeit suspendiertes Vanadiumpentoxid mit einer Lösung eines Silbersalzes, gegebenenfalls unter Zusatz eines Salzes des Metalls M erhitzt und isoliert.

10. Verfahren nach Anspruch 9, **dadurch gekennzeichnet, daß** man als Flüssigkeit Wasser verwendet.

11. Verfahren nach Anspruch 9, **dadurch gekennzeichnet, daß** man das Multimetalloxid durch Sprühtrocknen oder Abfiltrieren und Trocknen isoliert.

12. Verwendung von Multimetalloxiden gemäß Anspruch 1 zur Herstellung von Präkatalysatoren und Katalysatoren für die Gasphasenpartialoxidation von aromatischen Kohlenwasserstoffen.

13. Präkatalysator für die Herstellung oder Erzeugung von Schalenkatalysatoren für die Gasphasenpartialoxidation von aromatischen Kohlenwasserstoffen mit einem molekularen Sauerstoff enthaltenden Gas, bestehend aus einem inerten, nicht-porösen Trägermaterial und einer oder mehreren darauf schalenförmig aufgebrachten Schichten, wobei diese schalenförmige Schicht oder Schichten ein Multimetalloxid gemäß Anspruch 1 enthalten.

14. Präkatalysator gemäß Anspruch 13, der 30 bis 100 Gew.-% eines Multimetalloxids gemäß Anspruch 1, bezogen auf das Gesamtgewicht der schalenförmig aufgebrachten Schicht oder Schichten, enthält.

15. Präkatalysator gemäß Anspruch 13, dessen inertes, nicht-poröses Trägermaterial aus Steatit besteht.

16. Präkatalysator gemäß Anspruch 13, der in seiner schalenförmigen Schicht 30 bis 100 Gew.-%, bezogen auf das Gesamtgewicht dieser Schicht, eines Multimetalloxids gemäß Anspruch 2 enthält.

17. Präkatalysator gemäß Anspruch 13, der in seiner schalenförmigen Schicht 30 bis 100 Gew.-% eines Multimetalloxids gemäß Anspruch 3 enthält.

18. Präkatalysator gemäß Anspruch 13, der in seiner schalenförmigen Schicht 30 bis 100 Gew.-%, bezogen auf das Gesamtgewicht dieser Schicht, eines Multimetalloxids gemäß Anspruch 6 enthält.

19. Schalenkatalysator für die Gasphasenpartialoxidation von aromatischen Kohlenwasserstoffen mit einem molekularen Sauerstoff enthaltenden Gas, bestehend aus einem Katalysatorträger aus einem inerten, nicht-porösen Trägermaterial und einer oder mehreren darauf aufgebrachten, die katalytisch aktive Masse enthaltende, schalenförmige Schicht oder Schichten, dessen katalytisch aktive Masse, bezogen auf ihr Gesamtgewicht, 30 bis 100 Gew.-% eine oder mehrere Silber-Vanadiumoxid-Bronzen mit einem Ag : V-Atomverhältnis von 0,15 bis 0,95 enthält und eine BET-Oberfläche von 2 bis 100 m²/g hat, und weiter **dadurch gekennzeichnet, daß** der unter Verwendung eines Multimetalloxids gemäß Anspruch 1 hergestellt worden ist.

20. Schalenkatalysator gemäß Anspruch 19, der aus einem Präkatalysator gemäß Anspruch 13 hergestellt worden ist.

21. Verfahren zur Herstellung von Carbonsäuren und/oder Carbonsäureanhydriden durch die partielle Oxidation von aromatischen Kohlenwasserstoffen in der Gasphase mit einem molekularen Sauerstoff enthaltenden Gas bei erhöhter Temperatur an einem Katalysator, dessen katalytisch aktive Masse auf einem Katalysatorträger aus einem inerten, nicht-porösen Trägermaterial schalenförmig aufgebracht ist, **dadurch gekennzeichnet, daß** man einen Schalenkatalysator, dessen katalytisch aktive Masse, bezogen auf ihr Gesamtgewicht, 30 bis 100 Gew.-% eine oder mehrere Silber-Vanadiumoxid-Bronzen mit einem Ag:V-Atomverhältnis von 0,15 bis 0,95 enthält und eine BET-Oberfläche von 2 bis 100 m²/g hat, in An- oder Abwesenheit mindestens eines von diesem verschiedenen Schalenkatalysators zur Oxidation von aromatischen Kohlenwasserstoffen zu Carbonsäuren und/oder Carbonsäureanhydriden, der in seiner katalytisch aktiven Masse als wesentliche katalytisch aktive Bestandteile Vanadiumpentoxid und Anatas enthält, verwendet, und bei Anwesenheit eines solchen zweiten Schalenkatalysators, diesen in einer kombinierten Katalysatorschüttung mit dem Schalenkatalysator obenstehender Zusammensetzung im Oxidationsreaktor einsetzt, und **dadurch gekennzeichnet, daß** der ersten Schalenkatalysator, in situ im Oxidationsreaktor aus einem Präkatalysator gemäß Anspruch 13 erzeugt worden ist.

## Claims

1. A multimetal oxide of the formula I
Ag_{a-b}M_{b}V₂Oₓ * c H₂O, I
where M is a metal selected from the group consisting of Li, Na, K, Rb, Cs, Tl, Mg, Ca, Sr, Ba, Cu, Zn, Cd, Pb, Cr, Au, Al, Fe, Co, Ni and/or Mo,
a is from 0.3 to 1.9 and
b is from 0 to 0.5, with the proviso that the difference (a-b) ≥ 0.1 and
c is from 0 to 20 and
x is a number determined by the valence and amount of elements different from oxygen in the formula I,
which has a crystal structure giving an X-ray powder diffraction pattern which displays reflections at the lattice spacings d of 15.23 ± 0.6, 12.16 ± 0.4, 10.68 ± 0.3, 3.41 ± 0.04 , 3.09 ± 0.04, 3.02 ± 0.04 , 2.36 ± 0.04 and 1.80 ± 0.04 Å.

2. A multimetal oxide as claimed in claim 1 which has a fibrous crystal morphology having a mean ratio of fiber diameter to fiber length of less than 0.6.

3. A multimetal oxide as claimed in claim 1 which has a specific surface area determined by the BET method of from 3 to 250 m²/g.

4. A multimetal oxide as claimed in claim 1 in which a is from 0.5 to 1.0, b is from 0 to 0.3 and c is from 0 to 5.

5. A multimetal oxide as claimed in claim 1 in which a is from 0.6 to 0.9, b is from 0 to 0.1 and c is from 0 to 1.

6. A multimetal oxide as claimed in claim 1 and having the formula
AgₐV₂Oₓ* c H₂O,
where a is from 0.6 to 0.9, x is as defined in claim 1 and c is from 0 to 5.

7. A multimetal oxide as claimed in claim 1 whose X-ray powder diffraction pattern displays the following 17 reflections at the specified lattice spacings d [Å]:
| Reflections | d [Å] |
|---|---|
| 1 | 15.23 ± 0.6 |
| 2 | 12.16 ± 0.4 |
| 3 | 10.68 ± 0.3 |
| 4 | 5.06 ± 0.06 |
| 5 | 4.37 ± 0.04 |
| 6 | 3.86 ± 0.04 |
| 7 | 3.41 ± 0.04 |
| 8 | 3.09 ± 0.04 |
| 9 | 3.02 ± 0.04 |
| 10 | 2.58 ± 0.04 |
| 11 | 2.48 ± 0.04 |
| 12 | 2.42 ± 0.04 |
| 13 | 2.36 ± 0.04 |
| 14 | 2.04 ± 0.04 |
| 15 | 1.93 ± 0.04 |
| 16 | 1.80 ± 0.04 |
| 17 | 1.55 ± 0.04 |

8. A multimetal oxide as claimed in claim 7 whose reflections 1 to 17 have the following approximate relative intensities (Iᵣₑₗ [%]):
| Reflections | Iᵣₑₗ[%] |
|---|---|
| 1 | 16 |
| 2 | 11 |
| 3 | 18 |
| 4 | 11 |
| 5 | 23 |
| 6 | 16 |
| 7 | 80 |
| 8 | 61 |
| 9 | 100 |
| 10 | 23 |
| 11 | 24 |
| 12 | 23 |
| 13 | 38 |
| 14 | 26 |
| 15 | 31 |
| 16 | 43 |
| 17 | 36 |

9. A process for preparing multimetal oxides as claimed in claim 1, which comprises heating vanadium pentoxide suspended in a liquid with a solution of a silver salt, with or without addition of a salt of the metal M, and isolating the product.

10. A process as claimed in claim 9, wherein the liquid used is water.

11. A process as claimed in claim 9, wherein the multimetal oxide is isolated by spray drying or filtering off and drying.

12. The use of multimetal oxides as claimed in claim 1 for producing precatalysts and catalysts for the gas-phase partial oxidation of aromatic hydrocarbons.

13. A precatalyst for producing coated catalysts for the gas-phase partial oxidation of aromatic hydrocarbons by means of a gas comprising molecular oxygen, comprising an inert, nonporous support material and one or more layers applied thereto in the form of a shell or shells, wherein this/these shell-like layer or layers comprises/comprise a multimetal oxide as claimed in claim 1.

14. A precatalyst as claimed in claim 13 which comprises from 30 to 100% by weight of a multimetal oxide as claimed in claim 1, based on the total weight of the layer or layers applied in the form of a shell or shells.

15. A precatalyst as claimed in claim 13 whose inert, nonporous support material comprises steatite.

16. A precatalyst as claimed in claim 13 whose shell-like layer comprises from 30 to 100% by weight, based on the total weight of this layer, of a multimetal oxide as claimed in claim 2.

17. A precatalyst as claimed in claim 13 whose shell-like layer comprises from 30 to 100% by weight of a multimetal oxide as claimed in claim 3.

18. A precatalyst as claimed in claim 13, whose shell-like layer comprises from 30 to 100% by weight, based on the total weight of this layer, of a multimetal oxide as claimed in claim 6.

19. A coated catalyst for the gas-phase partial oxidation of aromatic hydrocarbons by means of a gas comprising molecular oxygen, comprising a catalyst support comprising an inert, nonporous support material and, applied thereto, one or more layer or layers comprising a catalytically active composition which comprises, based on its total weight, from 30 to 100% by weight of one or more silver-vanadium oxide bronzes having an Ag : V atomic ratio of from 0.15 to 0.95 and has a BET surface area of from 2 to 100 m²/g, and further a coated catalyst which has been produced using a multimetal oxide as claimed in claim 1.

20. A coated catalyst as claimed in claim 19 which has been produced from a precatalyst as claimed in claim 13.

21. A process for preparing carboxylic acids and/or carboxylic anhydrides by partial oxidation of aromatic hydrocarbons in the gas phase by means of a gas comprising molecular oxygen at elevated temperature over a catalyst whose catalytically active composition is applied in the form of a shell to a catalyst support comprising an inert, nonporous support material, wherein the catalyst used is a coated catalyst whose catalytically active composition, based on its total weight, comprises from 30 to 100% by weight of one or more silver-vanadium oxide bronzes having an Ag:V atomic ratio of from 0.15 to 0.95 and has a BET surface area of from 2 to 100 m²/g, in the presence or absence of at least one coated catalyst for the oxidation of aromatic hydrocarbons to carboxylic acids and/or carboxylic anhydrides which is different from the above-described coated catalyst and whose catalytically active composition comprises vanadium pentoxide and anatase as significant catalytically active constituents and, if such a second coated catalyst is present, it is used in a combined catalyst bed with the coated catalyst of the above composition in the oxidation reactor, and wherein the first coated catalyst has been produced in situ in the oxidation reactor from a precatalyst as claimed in claim 13.

## Revendications

1. Oxyde polymétallique répondant à la formule générale I
Ag_{a-b}M_{b}V₂Oₓ*c H₂O, I
dans laquelle
M représente un métal choisi parmi le groupe comprenant Li, Na, K, Rb, Cs, Tl, Mg, Ca, Sr, Ba, Cu, Zn, Cd, Pb, Cr, Au, Al, Fe, Co, Ni et/ou Mo,
a possède une valeur de 0,3 à 1,9 et
b possède une valeur de 0 à 0,5, avec cette mesure que la différence (a-b) est ≥ 0,1 et
c possède une valeur de 0 à 20 et
x représente un nombre qui est déterminé par la valence et par la fréquence des éléments différents de l'oxygène dans la formule I,
qui est présent dans une structure cristalline qui fournit un diagramme de diffractométrie à rayons X pour les substances pulvérulentes qui possède des indices de réfraction aux écartements des plans d 15,23 ± 0,6, 12,16 ± 0,4, 10,68 ± 0,3, 3,41 ± 0,04, 3,09 ± 0,04, 3,02 ± 0,04, 2,36 ± 0,04 et 1,80 ± 0,04 Å.

2. Oxyde polymétallique selon la revendication 1, qui présente une morphologie cristalline de forme fibreuse possédant un rapport moyen du diamètre des fibres à la longueur des fibres inférieur à 0,6.

3. Oxyde polymétallique selon la revendication 1, qui possède une surface spécifique selon BET de 3 à 250 m²/g.

4. Oxyde polymétallique selon la revendication 1, dans lequel a possède une valeur de 0,5 à 1,0, b possède une valeur de 0 à 0,3 et c possède une valeur de 0 à 5.

5. Oxyde polymétallique selon la revendication 1, dans lequel a possède une valeur de 0,6 à 0,9, b possède une valeur de 0 à 0,1 et c possède une valeur de 0 à 1.

6. Oxyde polymétallique selon la revendication 1, répondant à la formule
AgₐV₂Oₓ*c H₂O,
dans laquelle a possède une valeur de 0,6 à 0,9, x a la signification indiquée à la revendication 1 et c possède une valeur de 0 à 5.

7. Oxyde polymétallique selon la revendication 1, dont le diagramme de diffractométrie à rayons X pour les substances pulvérulentes présente les 17 indices de réfraction ci-après aux écartements des plans d [Å]:
| Indices de réfraction | d [Å] |
|---|---|
| 1 | 15,23 ± 0,6 |
| 2 | 12,16 ± 0,4 |
| 3 | 10,68 ± 0,3 |
| 4 | 5,06± 0,06 |
| 5 | 4,37 ± 0,04 |
| 6 | 3,86 ± 0,04 |
| 7 | 3,41 ± 0,04 |
| 8 | 3,09 ± 0,04 |
| 9 | 3,02 ± 0,04 |
| 10 | 2,58±0,04 |
| 11 | 2,48±0,04 |
| 12 | - 2,42 ± 0,04 |
| 13 | 2,36 ± 0,04 |
| 14 | 2,04 ± 0,04 |
| 15 | 1,93 ± 0,04 |
| 16 | 1,80 ± 0,04 |
| 17 | 1,55 ± 0,04 |

8. Oxyde polymétallique selon la revendication 7, dont les indices de réfraction 1 à 17 possèdent les intensités relatives approximatives (Iᵣₑₗ [%]) ci-après :
| Indices de réfraction | Iᵣₑₗ [%] |
|---|---|
| 1 | 16 |
| 2 | 11 |
| 3 | 18 |
| 4 | 11 |
| 5 | 23 |
| 6 | 16 |
| 7 | 80 |
| 8 | 61 |
| 9 | 100 |
| 10 | 23 |
| 11 | 24 |
| 12 | 23 |
| 13 | 38 |
| 14 | 26 |
| 15 | 31 |
| 16 | 43 |
| 17 | 36 |

9. Procédé pour la préparation d'oxyde polymétallique selon la revendication 1, **caractérisé en ce qu'**on chauffe du pentoxyde de vanadium mis en suspension dans un liquide avec une solution d'un sel d'argent, le cas échéant en ajoutant un sel du métal M, et on l'isole.

10. Procédé selon la revendication 9, **caractérisé en ce qu'**on utilise de l'eau à titre de liquide.

11. Procédé selon la revendication 9, **caractérisé en ce qu'**on isole l'oxyde polymétallique par séchage par pulvérisation ou bien par séparation par filtration et séchage.

12. Utilisation d'oxydes polymétalliques selon la revendication 1 pour la préparation de précatalyseurs et de catalyseurs pour l'oxydation partielle en phase gazeuse d'hydrocarbures aromatiques.

13. Précatalyseur pour la préparation ou l'obtention de catalyseurs enrobés pour l'oxydation partielle en phase gazeuse d'hydrocarbures aromatiques avec un gaz contenant de l'oxygène moléculaire, constitué par une matière de support inerte non poreuse et par une ou plusieurs couches appliquées en forme de coquille sur ladite matière de support, cette couche ou ces couches en forme de coquille contenant un oxyde polymétallique selon la revendication 1.

14. Précatalyseur selon la revendication 13, qui contient un oxyde polymétallique selon la revendication 1 à concurrence de 30 à 100 % en poids rapportés au poids total de la couche ou des couches appliquées en forme de coquille.

15. Précatalyseur selon la revendication 13, dont la matière de support inerte non poreuse est constituée de stéatite.

16. Précatalyseur selon la revendication 13 qui contient, dans sa couche en forme de coquille, un oxyde polymétallique selon la revendication 2 à concurrence de 30 à 100 % en poids rapportés au poids total de cette couche.

17. Précatalyseur selon la revendication 13, qui contient, dans sa couche en forme de coquille, un oxyde polymétallique selon la revendication 3, à concurrence de 30 à 100 % en poids.

18. Précatalyseur selon la revendication 13 qui contient, dans sa couche en forme de coquille, un oxyde polymétallique selon la revendication 6 à concurrence de 30 à 100 % en poids rapportés au poids total de cette couche.

19. Catalyseur enrobé pour l'oxydation partielle en phase gazeuse d'hydrocarbures aromatiques avec un gaz contenant de l'oxygène moléculaire, constitué par un support de catalyseur constitué d'une matière de support inerte non poreuse et par une ou plusieurs couches en forme de coquille, contenant la matière active du point de vue catalytique, appliquées sur ladite matière de support, dont la matière active du point de vue catalytique contient, à concurrence de 30 à 100 % en poids rapportés à son poids total, un ou plusieurs bronzes d'oxyde d'argent-vanadium possédant un rapport atomique Ag: V de 0,15 à 0,95 et une surface selon BET de 2 à 100 m²/g, et **caractérisé en outre en ce qu'**il a été préparé en utilisant un oxyde polymétallique selon la revendication 1.

20. Catalyseur enrobé selon la revendication 19, qui a été préparé à partir d'un précatalyseur selon la revendication 13.

21. Procédé pour la préparation d'acides carboxyliques et/ou d'anhydrides d'acides carboxyliques par l'oxydation partielle d'hydrocarbures aromatiques en phase gazeuse avec un gaz contenant de l'oxygène moléculaire, à une température élevée, sur un catalyseur dont la matière active du point de vue catalytique a été appliquée en forme de coquille sur un support de catalyseur constitué d'une matière de support inerte non poreuse, **caractérisé en ce qu'**on utilise un catalyseur enrobé dont la matière active du point de vue catalytique contient, à concurrence de 30 à 100 % en poids rapportés à son poids total, un ou plusieurs bronzes d'oxyde d'argent-vanadium possédant un rapport atomique Ag : V de 0,15 à 0,95 et possède une surface selon BET de 2 à 100 m²/g, en présence ou en l'absence d'au moins un catalyseur enrobé différent du premier cité pour l'oxydation d'hydrocarbures aromatiques donnant lieu à des acides carboxyliques et/ou à des anhydrides d'acides carboxyliques, qui contient, dans sa matière active du point de vue catalytique, à titre de constituants essentiels actifs du point de vue catalytique, du pentoxyde de vanadium et de l'anatase, et, en présence d'un deuxième catalyseur enrobé de ce type, on met en oeuvre ce dernier, sous forme d'un lit de catalyseur combiné, avec le catalyseur enrobé possédant la composition indiquée ci-dessus, dans le réacteur d'oxydation, et **caractérisé en ce que** le premier catalyseur enrobé a été généré in situ dans le réacteur d'oxydation à partir d'un précatalyseur selon la revendication 13.
